# EUROPEAN PATENT APPLICATION

(11) **EP 3 984 597 A1**
(43) Date of publication of application: **20.04.2022**
(21) Application number: 20840275.0
(22) Date of filing: 10.07.2020
(51) Int. Cl.: A61P 9/00, A61P 9/04, A61P 9/10, A61P 43/00, A61K 31/704, A61K 35/15

(54) **DEVELOPMENT OF THERAPY FOR IMPROVING MYOCARDIAL CONTRACTION AND METHOD FOR INHIBITING CARDIOMYOCYTE DEATH**

(30) Priority: 12.07.2019 JP 2019130571
(71) Applicant: Kyushu University, National University Corporation, Nishi-ku Fukuoka-shi Fukuoka 819-0395 (JP)
(72) Inventor: TSUTSUI, Hiroyuki, Fukuoka-shi, Fukuoka 819-0395 (JP); IDE, Tomomi, Fukuoka-shi, Fukuoka 819-0395 (JP); OHTANI, Kisho, Fukuoka-shi, Fukuoka 819-0395 (JP); MATSUSHIMA, Shoji, Fukuoka-shi, Fukuoka 819-0395 (JP); IKEDA, Masataka, Fukuoka-shi, Fukuoka 819-0395 (JP)
(74) Representative: Hoffmann Eitle
(86) International application number: PCT/JP2020/027115
(87) International publication number: WO 2021/010344

(57) **Abstract**

The present invention provides a pharmaceutical for preventing and/or treating non-ischemic cardiomyopathy, the pharmaceutical comprising dendritic cells obtained by a method comprising a step of culturing mononuclear cells in the presence of GM-CSF and IL-2 and a step of pulsing the cultured cells with α-galactosylceramide.

## Description

### Technical Field

The present invention relates to a pharmaceutical for preventing and/or treating cardiomyopathy, the pharmaceutical comprising dendritic cells pulsed with α-galactosylceramide (α-GalCer), and a method of producing the same.

### Background Art

Cardiomyopathy is generally defined as a myocardial disease associated with cardiac dysfunction and has main symptoms such as cardiac hypertrophy, cardiac dilatation, and a decrease in the contractile function and/or diastolic function. The cardiomyopathy is classified into dilated cardiomyopathy, hypertrophic cardiomyopathy, restrictive cardiomyopathy, arrhythmogenic (proarrhythmic) right ventricular cardiomyopathy, unclassifiable cardiomyopathy, and specific cardiomyopathy. The specific cardiomyopathy includes ischemic cardiomyopathy, valvular cardiomyopathy, hypertensive cardiomyopathy, inflammatory cardiomyopathy, metabolic cardiomyopathy, and the like.

The cause of cardiomyopathy is not clear in many cases, and known causes include genetic factors, immune abnormalities, viral infections, and environmental factors. In the classified cardiomyopathy, hypertrophic cardiomyopathy is defined as "a group of diseases characterized by (1) hypertrophy of the left or right ventricular myocardium and (2) a decrease in left ventricular diastolic function due to cardiac hypertrophy", and dilated cardiomyopathy is defined as "a group of diseases characterized by left ventricular dilatation and left ventricular contractile dysfunction, without complications of abnormal stress conditions (high blood pressure and valvular disease) and coronary artery disease that can cause diffuse systolic dysfunction". Myocardial infarction and angina due to arteriosclerosis may be associated with ischemic cardiomyopathy. In addition, arrhythmias including atrial fibrillation may be associated with cardiomyopathies such as arrhythmogenic right ventricular cardiomyopathy. In the treatment of patients who develop secondary cardiomyopathy caused by other diseases, the treatment of the causative disease may lead to recovery of myocardial function.

Further, the risk of developing cardiomyopathy (drug-induced cardiomyopathy) is known to be increased in a dose-dependent manner in a case where an anthracycline-based anticancer agent such as doxorubicin is administered to a cancer patient (Non-Patent Literatures 1 and 2). Once cardiomyopathy develops, the prognosis is extremely unsatisfactory and chronic heart failure may develop so that cancer treatment may be difficult to continue in some cases. The mechanism of myocardial dysfunction caused by doxorubicin is still unknown, and no effective treatment method has been known so far. It has been reported that the expression level of inflammatory cytokines is increased and the infiltration of inflammatory cells is also increased in the myocardium of patients with drug-induced cardiomyopathy caused by doxorubicin (Non-Patent Literatures 3 and 4).

### Citation List

### Patent Literature

[Patent Literature 1] WO 2015/129791
[Patent Literature 2] WO 2003/016326

### Non Patent Literature

[Non-Patent Literature 1] Barrett P J et al. Ann. Oncol. 2009; 20: 816-827.
[Non-Patent Literature 2] Felker GM et al. N. Eng. J. Med. 2000; 342: 1077-1084.
[Non-Patent Literature 3] Wang L et al. Sci. Rep. 2016 Jun 21; 6: 28399.
[Non-Patent Literature 4] A. Riad et al. European Journal of Heart Failure 10 (2008) 233-243.
[Non-Patent Literature 5] Sobirin M.A. et al., Circ. Res. 2012; 111: 1037-1047.
[Non-Patent Literature 6] Van Kaer L. Nat. Rev. Immunol, 2005; 5: 31-42.
[Non-Patent Literature 7] Ishikawa E. et al., Int. J. Cancer 2005; 117: 265-273.
[Non-Patent Literature 8] Fujii et al., Nat. Immunol. 3: 867 (2002).
[Non-Patent Literature 9] Ishikawa A. et al., Clin. Cancer Res. 2005; 11: 1910-1917.
[Non-Patent Literature 10] Godfrey DI, et al., Nat. Rev. Immunol. 2007; 7: 505-518.
[Non-Patent Literature 11] Homma T. et al., J. Mol. Cell. Cardiol. 2013; 62: 179-188.
[Non-Patent Literature 12] Endre Pal, et al., J. Immunol. 2001; 166: 662-668.
[Non-Patent Literature 13] Sharif S, et al., Nat. Med. 2001; 7: 1057-1062.
[Non-Patent Literature 14] Morimoto et al., Circulation Research, 2017, 185-194.
[Non-Patent Literature 15] Nonaka et al., British Journal of Pharmacology 2015, 172, 2369-2382.

### Summary of Invention

### Technical Problem

The present inventors found that administration of α-GalCer has been effective in prevention or treatment of myocardial infarction or complications (such as heart failure, arrhythmia, papillary muscle rupture, cardiac rupture, ventricular aneurysm, and post-myocardial infarction syndrome) thereof and ischemia-reperfusion injury (Patent Literature 1 and Non-Patent Literature 5).

Therefore, an object of the present invention is to provide a pharmaceutical for preventing and/or treating cardiomyopathy and a method of preventing and/or treating cardiomyopathy. Further, another object of the present invention is to provide a pharmaceutical for improving the myocardial contractility, a pharmaceutical for inhibiting and/or preventing myocardial cell death, a pharmaceutical for inhibiting and/or improving myocardial atrophy, a pharmaceutical for progress-inhibiting and/or treating myocardial fibrosis, or a pharmaceutical for inhibiting myocardial inflammation.

### Solution to Problem

α-Galactosylceramide (α-GalCer) is a glycosphingolipid isolated from a sponge and is known to activate natural killer T cells (NKT cells) (Non-Patent Literature 6). The activation of NKT cells by α-GalCer is transient, and various examinations such as application of dendritic cells pulsed with α-GalCer have been performed in order to solve the problem of a decrease in drug efficacy and liver damage caused when repeated administration is performed (Patent Literature 2 and Non-Patent Literatures 7 to 9).

NKT cells express only one type of T cell receptor (TCR), are activated by recognizing only glycolipid antigens presented by CD1d molecule, and produce various cytokines. The T cell receptor has an invariable α-chain and is referred to as Vα14-Jα18 in mice and Vα24-Jα18 in humans. The activated NKT cells rapidly produce large amounts of T helper (Th1) type 1-cytokines of which representatives are interferon (IFN)-γ and tumor necrosis factor (TNF)-α, or Th2-type cytokines of which representatives are interleukins (IL)-4 and IL-10 and form acquired immune response. In this manner, NKT cells are thought to function as a bridge between the innate immunity and the acquired immunity to control tissue inflammation (Non-Patent Literature 10).

Since NKT cells produce both Th1 and Th2-type cytokines, control of the balance of the immune response of NKT cells is important in cellular immunotherapy using NKT cells (Non-Patent Literatures 5 and 11 to 13). For example, in a case of treating an autoimmune disease, the immune response of NKT cells is shifted to the Th2-type to activate humoral immunity. Further, in a case of treating a disease such as a specific cancer or an infectious disease, the immune response of NKT cells is shifted to the Th1-type to activate cell-mediated immunity.

As a result of intensive examination conducted by the present inventors in order to solve the above-described problems, it was found that NKT cells are activated, myocardial contractility is improved, and thus myocardial cell death can be inhibited by administering dendritic cells pulsed with α-GalCer.

That is, the present invention provides the following [1] to [34].
[1] A pharmaceutical for preventing and/or treating non-ischemic cardiomyopathy, the pharmaceutical comprising dendritic cells obtained by a method comprising a step of culturing mononuclear cells in the presence of GM-CSF and IL-2 and a step of pulsing the cultured cells with α-galactosylceramide.
[2] The pharmaceutical according to [1], in which the non-ischemic cardiomyopathy is dilated cardiomyopathy or drug-induced cardiomyopathy caused by an anthracycline-based anticancer agent.
[3] The pharmaceutical according to [1] or [2], further comprising an anthracycline-based anticancer agent.
[4] The pharmaceutical according to any one of [1] to [3], in which the pharmaceutical is used for preventing and/or treating cardiomyopathy of a subject to whom an anthracycline-based anticancer agent has been administered.
[5] A method of producing a pharmaceutical for preventing and/or treating non-ischemic cardiomyopathy, the method comprising a step of culturing mononuclear cells in the presence of GM-CSF and IL-2; and a step of pulsing the cultured cells with α-galactosylceramide.
[6] The method of producing a pharmaceutical according to [5], in which the non-ischemic cardiomyopathy is dilated cardiomyopathy or drug-induced cardiomyopathy caused by an anthracycline anticancer agent.
[7] A method of preventing and/or treating non-ischemic cardiomyopathy, the method comprising administering a pharmaceutical that contains dendritic cells obtained by a method comprising a step of culturing mononuclear cells in the presence of GM-CSF and IL-2 and a step of pulsing the cultured cells with α-galactosylceramide, to a subject in need thereof.
[8] The method for preventing and/or treating non-ischemic cardiomyopathy according to [7], in which the non-ischemic cardiomyopathy is drug-induced cardiomyopathy caused by an anthracycline-based anticancer agent or dilated cardiomyopathy.
[9] The method for preventing and/or treating non-ischemic cardiomyopathy according to [7] or [8], in which the subject is a patient to whom an anthracycline-based anticancer agent has been administered.
[10] A pharmaceutical for inhibiting a decrease in myocardial contractility, comprising dendritic cells obtained by a method comprising a step of culturing mononuclear cells in the presence of GM-CSF and IL-2 and a step of pulsing the cultured cells with α-galactosylceramide.
[11] A method of inhibiting a decrease in myocardial contractility, the method comprising: administering a pharmaceutical comprising dendritic cells obtained by a method comprising a step of culturing mononuclear cells in the presence of GM-CSF and IL-2 and a step of pulsing the cultured cells with α-galactosylceramide, to a subject in need thereof.
[12] The method according to [11], in which the subject is a patient suffering from non-ischemic cardiomyopathy.
[13] The method according to [11], in which the subject is a patient to whom an anthracycline-based anticancer agent has been administered.
[14] A pharmaceutical for inhibiting or preventing myocardial cell death, the pharmaceutical comprising dendritic cells obtained by a method comprising a step of culturing mononuclear cells in the presence of GM-CSF and IL-2 and a step of pulsing the cultured cells with α-galactosylceramide.
[15] A method of inhibiting or preventing myocardial cell death, the method comprising: administering a pharmaceutical comprising dendritic cells obtained by a method comprising a step of culturing mononuclear cells in the presence of GM-CSF and IL-2 and a step of pulsing the cultured cells with α-galactosylceramide to a subject in need thereof.
[16] The method according to [15], in which the subject is a patient suffering from non-ischemic cardiomyopathy.
[17] The method according to [15], in which the subject is a patient to whom an anthracycline-based anticancer agent has been administered.
[18] A pharmaceutical for inhibiting or improving myocardial atrophy, the pharmaceutical comprising: dendritic cells obtained by a method comprising a step of culturing mononuclear cells in the presence of GM-CSF and IL-2 and a step of pulsing the cultured cells with α-galactosylceramide.
[19] A method of inhibiting or improving myocardial atrophy, the method comprising: administering a pharmaceutical comprising dendritic cells obtained by a method comprising a step of culturing mononuclear cells in the presence of GM-CSF and IL-2 and a step of pulsing the cultured cells with α-galactosylceramide to a subject in need thereof.
[20] The method according to [19], in which the subject is a patient suffering from non-ischemic cardiomyopathy.
[21] A pharmaceutical for progress-inhibiting and/or treating myocardial fibrosis (particularly, fibrosis of myocardial interstitium), the pharmaceutical comprising: dendritic cells obtained by a method comprising a step of culturing mononuclear cells in the presence of GM-CSF and IL-2 and a step of pulsing the cultured cells with α-galactosylceramide.
[22] A method of progress-inhibiting or treating myocardial fibrosis (particularly, fibrosis of myocardial interstitium), the method comprising: administering a pharmaceutical comprising dendritic cells obtained by a method comprising a step of culturing mononuclear cells in the presence of GM-CSF and IL-2 and a step of pulsing the cultured cells with α-galactosylceramide to a subject in need thereof.
[23] The method according to [22], in which the subject is a patient suffering from non-ischemic cardiomyopathy.
[24] The method according to [22], in which the subject is a patient to whom an anthracycline anticancer agent has been administered.
[25] A pharmaceutical for inhibiting myocardial inflammation, comprising: dendritic cells obtained by a method comprising a step of culturing mononuclear cells in the presence of GM-CSF and IL-2 and a step of pulsing the cultured cells with α-galactosylceramide.
[26] A method of inhibiting myocardial inflammation, comprising: administering a pharmaceutical comprising dendritic cells obtained by a method comprising a step of culturing mononuclear cells in the presence of GM-CSF and IL-2 and a step of pulsing the cultured cells with α-galactosylceramide to a subject in need thereof.
[27] Use for preventing and/or treating non-ischemic cardiomyopathy of dendritic cells obtained by a method comprising a step of culturing mononuclear cells in the presence of GM-CSF and IL-2 and a step of pulsing the cultured cells with α-galactosylceramide.
[28] The use according to [27], in which the non-ischemic cardiomyopathy is dilated cardiomyopathy or drug-induced cardiomyopathy caused by an anthracycline anticancer agent.
[29] Use for inhibiting a decrease in myocardial contractility of dendritic cells obtained by a method comprising a step of culturing mononuclear cells in the presence of GM-CSF and IL-2 and a step of pulsing the cultured cells with α-galactosylceramide.
[30] The use according to [29], in which the non-ischemic cardiomyopathy is dilated cardiomyopathy or drug-induced cardiomyopathy caused by an anthracycline anticancer agent.
[31] Use for inhibiting or preventing myocardial cell death of dendritic cells obtained by a method comprising a step of culturing mononuclear cells in the presence of GM-CSF and IL-2 and a step of pulsing the cultured cells with α-galactosylceramide.
[32] Use for inhibiting or improving myocardial atrophy of dendritic cells obtained by a method comprising a step of culturing mononuclear cells in the presence of GM-CSF and IL-2 and a step of pulsing the cultured cells with α-galactosylceramide.
[33] Use for progress-inhibiting and/or treating myocardial fibrosis (particularly, fibrosis of myocardial interstitium) of dendritic cells obtained by a method comprising a step of culturing mononuclear cells in the presence of GM-CSF and IL-2 and a step of pulsing the cultured cells with α-galactosylceramide.
[34] Use for inhibiting myocardial inflammation of dendritic cells obtained by a method comprising a step of culturing mononuclear cells in the presence of GM-CSF and IL-2 and a step of pulsing the cultured cells with α-galactosylceramide.

### Advantageous Effects of Invention

According to the present invention, it is possible to provide a pharmaceutical for preventing and/or treating cardiomyopathy, a method of preventing and/or treating cardiomyopathy, a method of inhibiting and/or improving a decrease in myocardial contractility, a method of inhibiting and/or preventing myocardial cell death, or a method of inhibiting and/or improving myocardial atrophy. Further, according to the present invention, it is possible to progress-inhibit and/or to treat myocardial fibrosis (particularly, fibrosis of myocardial interstitium). A decrease in myocardial contractility and myocardial fibrosis may be shown as symptoms associated with diseases other than cardiomyopathy. Therefore, the present invention is not limited to the decrease in myocardial contractility and the progression of myocardial fibrosis associated with cardiomyopathy and enables inhibition of a decrease in myocardial contractility and progression of myocardial fibrosis associated with other diseases. Furthermore, according to the present invention, myocardial inflammation can be inhibited.

α-GalCer is known to cause serious liver damage when systemically administered to humans. However, by administering dendritic cells pulsed with α-GalCer, the risk of side effects can be reduced and the effect of α-GalCer can be sustained for a longer period of time.

In addition, the contractile function of the heart is regulated by the β-receptor-cAMP (cyclic AMP)/PKA (protein kinase A)-pPLN (phosphorylated phospholamban)-SERCA (sarcoplasmic reticulum Ca²⁺ ATPase) pathway. According to the present invention, in a case where NKT cells are activated, the expression level of IFN-γ is increased, phosphorylation of PLN (phospholamban) is promoted, and thus a decrease in myocardial contractile function can be inhibited.

Further, according to the present invention, fibrosis of myocardial cells and myocardial cell death (apoptosis of myocardial cells) can be inhibited, and a cardioprotective effect (for example, an effect of inhibiting a decrease in cardiac function) can be exhibited.

### Brief Description of Drawings

FIG. 1 is a graph showing changes over time in body weight (BW) and left ventricular fractional shortening rate (FS) at each time point of Days 0, 7, 14, 21, and 28.
FIG. 2 is a graph showing the expression levels of Vα14Jα18 on Days 7 and 14.
FIG. 3 is a graph showing changes in inflammatory cytokines on Days 7 and 14.
FIG. 4(a) is a diagram showing a test method of evaluating the effect of αGalCer on DOX mice, and FIG. 4(b) is a graph comparing the expression levels of Vα14Jα18 in each group of mice.
FIG. 5 is a graph comparing the left ventricular fractional shortening rate (FS), the left ventricular end-diastolic dimension (LVDd), and the left ventricular end-systolic dimension (LVDs) of each group of mice.
FIG. 6 is a graph comparing the body weight (BW), the true lumen (TL) in the echocardiogram, the ratio of the whole heart to the true lumen (WH/TL), and the ratio of the left ventricle to the true lumen (LV/TL) of each group of mice.
FIG. 7 is a photograph taken by TUNEL-staining myocardial cells collected from each group of mice.
FIG. 8 is a graph comparing the expression levels of inflammatory cytokines in each group of mice on Days 7 and 14.
FIG. 9 is a graph comparing the expression levels of inflammatory cytokines in each group of mice on Days 7 and 14.
FIG. 10(a) is a diagram showing a test method of evaluating the effect of an anti-IFN-γ antibody, and FIG. 10(b) is a graph comparing the ejection fraction (EF), the left ventricular fractional shortening rate (FS), the left ventricular end-diastolic dimension (LVDd), and the left ventricular end-systolic dimension (LVDs) of each group of mice.
FIG. 11 is a graph comparing the expression levels of IFNγ in myocardial cells of each group of mice.
FIG. 12 is a graph showing changes in mRNA expression levels of Vα14Jα18 and inflammatory cytokines due to heterologous tissue transplantation.
FIG. 13 is a graph showing activation of NKT cells in a case where a suspension of α-GalCer/DC is administered once.
FIG. 14(a) is a photograph showing a cross section of a myocardial tissue of a DCM mouse and a wild-type Balb/c mouse (WT mouse), and FIG. 14(b) is a graph comparing the heart weight HW/BW and the lung weight LW/BW of both mice and a graph comparing the expression levels of Vα14Jα18 in the spleen.
FIG. 15 is a cytogram using spleen cells derived from DCM and WT mice.
FIG. 16(a) is a graph showing the survival rate of DCM mice over time, and FIG. 16(b) is a graph showing changes in left ventricular ejection fraction (LVEF) of 5-week-old (at the time of administration) and 9-week-old DCM mice.
FIG. 17(a) is a graph showing the left ventricular end-diastolic dimension (LVDd) of 5-week-old and 9-week-old mice and the heart weight and the lung weight of 9-week-old mice in four groups, FIG. 17(b) is a photograph showing a cross section of myocardial cells, and FIG. 17(c) is a graph showing the proportion of fibrotic cells of the myocardial interstitium in the four groups.
FIG. 18 is a graph comparing the expression levels of Vα14Jα18 and cytokines in the four groups.
FIG. 19 is a graph showing changes in left ventricular ejection fraction (LVEF) before and after administration of α-GalCer/DC or PBS in DCM mice.
FIG. 20 is a graph showing the expression levels of inflammatory cytokines.
FIG. 21 is a graph showing changes in expression levels of cytokines in blood.
FIG. 22(a) is a diagram showing a test method of evaluating the acute effect of α-GalCer/DC, and FIG. 22(b) is a graph showing the results thereof.
FIG. 23 is a graph showing changes in pPLN concentration due to α-GalCer/DC.
FIG. 24 is a photograph of electrophoresis showing changes in pPLN concentration in cultured myocardial cells.
FIG. 25 is a photograph of electrophoresis showing changes in p-Stat1 and pPLN concentrations in cultured myocardial cells.
FIG. 26 is a diagram showing the results of GO analysis of a microarray.
FIG. 27 is a graph showing the expression levels of mRNA in the myocardium of wild mice (WT) or DCM mice (DCM).
FIG. 28 is a western blot showing the expression levels of protein in the myocardium of wild mice (WT) or DCM mice (DCM) and a graph quantifying the expression levels.
FIG. 29 is a graph showing the expression levels of proteins Angpt1 and vegfa in the myocardium of wild mice (WT) or DCM mice (DCM).
FIG. 30 is a graph showing the expression level of each gene 4 days after administration of α-GalCer/DC to Balb/c mice.
FIG. 31 is a western blot showing the expression levels of protein in the myocardium and a graph quantifying the expression levels.
FIG. 32 is a Western blot showing the expression level of each protein in the myocardium 4 days after administration of α-GalCer alone or α-GalCer/DC to mice.
FIG. 33 is a western blot showing changes in the expression level of each protein over time and a graph quantifying the expression levels.
FIG. 34 is a graph showing the expression levels of cytokine genes in the myocardium.
FIG. 35 is a western blot showing the expression level of each protein.
FIG. 36 is a graph showing the expression levels of cytokine genes in the myocardium.
FIG. 37 is a western blot showing the expression levels of protein 28 days after administration of PBS or α-GalCer/DC.
FIG. 38 is a graph showing changes in expression levels of genes after administration of IFNγ, IL4, or IL10 to myocardial fibroblasts.
FIG. 39 is a western blot showing the expression levels of protein when TGFβ is administered to myocardial fibroblasts pretreated with IFNγ and a graph quantifying the expression levels.
FIG. 40 is a western blot showing the expression levels of protein when myocardial fibroblasts are treated with IFNγ and a graph quantifying the expression levels.
FIG. 41 is a western blot showing the expression levels of protein when myocardial fibroblasts are treated with IL4 or IL10 and a graph quantifying the expression levels.
FIG. 42(a) is a graph showing the expression levels of Angpt1 after administration of IFNγ, IL4, or IL10 to offspring rat myocardial cells. FIG. 42(B) is a western blot showing the expression levels of pStat1 and Stat1.
FIG. 43 is a graph showing changes in the expression levels of Angpt1 due to administration of IFNγ.
FIG. 44 is a western blot showing the expression levels of protein after administration of IFNγ to primary cultured myocardial cells and a graph quantifying the expression levels.
FIG. 45 is a western blot showing the expression levels of protein after administration of IL4 or IL10 to primary cultured myocardial cells and a graph quantifying the expression levels.
FIG. 46 is a graph showing the results of QOL evaluation using the Minnesota Living with Heart Failure Questionnaire (MLHFQ) or the Kansas City Cardiomyopathy Questionnaire (KCCQ).

### Description of Embodiments

Embodiments of the present invention will be described in detail below.

A first embodiment of the present invention relates to a pharmaceutical for preventing and/or treating cardiomyopathy, the pharmaceutical comprises dendritic cells obtained by a method comprising a step of culturing mononuclear cells in the presence of granulocyte macrophage colony-stimulating factor (GM-CSF) and IL-2 and a step of pulsing the cultured cells with α-GalCer.

The dendritic cells according to the present embodiment are dendritic cells obtained by a method comprising a step of culturing mononuclear cells in the presence of GM-CSF and IL-2 and a step of pulsing the cultured cells with α-GalCer.

The method of preparing the dendritic cells comprises a step of culturing mononuclear cells in the presence of GM-CSF and IL-2 (hereinafter, referred to as a culturing step) and a step of pulsing the cultured cells with α-GalCer (hereinafter, referred to as a pulsing step), and this method is described in, for example, Non-Patent Literatures 7 and 8.

The culturing step is a step of culturing the mononuclear cells separated according to a typical method in an appropriate medium containing GM-CSF and IL-2, and the mononuclear cells are differentiated and induced to dendritic cells by such a step. The mononuclear cells can be separated from various animal tissues, typically, for example, from peripheral blood or apheresis extracellular fluid by density gradient centrifugation.

In consideration of the safety in subsequent administration of dendritic cells, it is preferable that mononuclear cells are collected from animals of the same species or closely related species to the subject to which the cells are administered. For example, in a case where the subject is a human, cells collected from a human of the same species are preferably used, and cells collected from the human to which the cells are administered, that is, autologous mononuclear cells are more preferably used.

The medium used in the culturing step and the pulsing step described below is a medium usually used when dendritic cells are differentiated and induced from mononuclear cells, for example, an AIM-V medium or RPMI-1640 medium, and other components such as serum, plasma, or albumin may be added thereto as necessary. In the culturing step, these media to which GM-CSF is added such that the final concentration thereof is set to be in a range of 500 to 1000 U/mL and preferably approximately 800 U/mL and to which IL-2 is added such that the final concentration thereof is set to be in a range of 50 to 200 JRU/mL and preferably approximately 100 JRU/mL are used, and mononuclear cells are cultured for 5 to 10 days.

The pulsing step is a step of pulsing the dendritic cells prepared by the above-described culturing step with α-GalCer and is specifically performed by culturing the dendritic cells in a medium containing α-GalCer at a final concentration of 50 to 200 ng/mL and preferably approximately 100 ng/mL for 8 to 48 hours. The α-GalCer used in the pulsing step may be α-galactosylceramide itself or a salt thereof, an ester thereof, or a derivative thereof (for example, those described in Biosci. Biotechnol. Biochem., Tashiro T., 2012; 76 (6): 1055-67). α-GalCer can be purchased from Funakoshi Co., Ltd., Regimmune Co., Ltd., and the like.

The culturing step and the pulsing step may be carried out separately, or the culturing step and the pulsing step can be carried out at the same time by adding α-GalCer to the medium in the latter half of the culturing step.

Mononuclear cells and dendritic cells obtained by the culturing step may be cryopreserved according to a typical method, fused as necessary, and used in the subsequent steps. Similarly, the dendritic cells pulsed with α-GalCer obtained by the pulsing step can be cryopreserved after preparation, fused as necessary, and used as the pharmaceutical according to the present embodiment.

The dendritic cells pulsed with α-GalCer which have been prepared in the above-described manner activate NKT cells in the body of the subject to which the cells have been administered and induce or promote the production of IFN-γ. The activation of NKT cells gradually increases and can be maintained until the 7th day. The production of IFN-γ also gradually increases and can be maintained until the 28th day. In the myocardial cells, IFN-γ is thought to promote phosphorylation of PLN (phospholamban) and enhance the myocardial contractile function. Further, α-GalCer exhibits an effect of inhibiting a decrease in left ventricular ejection fraction (LVEF) associated with cardiomyopathy as well as an effect of inhibiting fibrosis of myocardial interstitium. The effects of the dendritic cells pulsed with α-GalCer can be confirmed by using an evaluation system that reflects the disease, for example, a disease model animal or disease model animal-derived cells. In a case where the morbidity or onset of a disease is prevented by dendritic cells, the condition of the disease is improved, or worsening of the condition of the disease is inhibited, it is determined that the dendritic cells have an effect of preventing and/or treating the disease. It is preferable that the evaluation system used here is an evaluation system for a disease that is the same as or related to the disease intended to be treated.

In a certain embodiment, the effect of dendritic cells pulsed with α-GalCer to prevent and/or treat a disease can be confirmed by collecting samples such as blood or organs from a subject to which dendritic cells pulsed with α-GalCer have been administered and measuring the amount of the inflammatory cytokines (such as IFN-γ) or biomarkers (such as plasma BNP (brain natriuretic peptide), serum NT-proBNP, plasma ANP, myocardial troponin, plasma noradrenaline, aldosterone, and plasma renin activity) or the expression level of the genes thereof. The effect thereof to prevent and/or treat the disease may also be confirmed by evaluating the cardiac function using echocardiography. Further, the effect thereof may be confirmed by collecting samples containing NKT cells from the subject in advance, bringing the samples into contact with dendritic cells pulsed with α-GalCer, and measuring the production amount of cytokines described above or the expression level of genes of the NKT cells. In a case where the amount of cytokines or the expression level of genes in the samples is increased by the dendritic cells pulsed with α-GalCer, the dendritic cells pulsed with α-GalCer are determined to be suitable for preventing and/or treating cardiomyopathy. The method of measuring the amount of cytokines or the expression level of genes can be carried out by an immunoassay, RT-PCR, or other methods known to those skilled in the art.

The pharmaceutical according to the present embodiment can be used for preventing and/or treating such a disease by administering the pharmaceutical to a subject who has suffered or may suffer from non-ischemic cardiomyopathy. In addition, the pharmaceutical according to the present embodiment may be used for preventing and/or treating cardiomyopathy-like symptoms associated with other diseases (for example, cardiovascular diseases).

In the present specification, the term "non-ischemic cardiomyopathy" indicates cardiomyopathy other than ischemic cardiomyopathy, and cardiovascular diseases caused by ischemic injury (myocardial infarction or complications thereof (for example, heart failure, arrhythmia, papillary muscle rupture, cardiac rupture, ventricular aneurysm, and post-myocardial infarction syndrome), stunning, hibernation, microcirculatory disorder, angina, and ischemia-reperfusion injury) are excluded. Examples of non-ischemic cardiomyopathy include dilated cardiomyopathy, hypertrophic cardiomyopathy, restrictive cardiomyopathy, arrhythmogenic right ventricular cardiomyopathy, and secondary cardiomyopathy. Non-ischemic cardiomyopathy may be hereditary cardiomyopathy or non-hereditary cardiomyopathy.

Examples of diseases that can cause secondary cardiomyopathy include autoimmune diseases (such as rheumatoid arthritis, polymyositis, SLE, and mixed connective tissue disease), inflammatory diseases (such as viral or non-viral myocarditis and sarcoidosis), infiltrative diseases (such as amyloidosis and hemochromatosis), endocrine diseases (such as hyperthyroidism, Cushing's disease, pheochromocytoma, adrenal insufficiency, and abnormal growth hormone secretion), metabolic diseases (such as diabetes), congenital enzyme abnormalities (such as Fabry's disease, Pompe disease, Harler's syndrome, and Hunter's syndrome), neuromuscular diseases (such as muscular dystrophy, laminopathy, and mitochondrial disease), hypertensive heart disease, valvular disease, atrial septal defect, eosinophilia endocarditis, endocardial elasticity, and arrhythmia (such as sick sinus syndrome, atrioventricular block, atrial fibrillation, and atrial tachycardia).

Examples of the drugs that can cause drug-induced cardiomyopathy include an anthracycline anticancer agent (such as doxorubicin, idarubicin, epirubicin, mitoxantrone, or liposomal anthracycline), an alkylating agent (such as cyclophosphamide or ifosfamide), an antimetabolic agent (such as clofarabine), a microtubule inhibitor (such as docetaxel or paclitaxel), a humanized monoclonal antibody (such as trastuzumab, bevacizumab, or pertuzumab), an immune checkpoint inhibitor (such as nivolumab or ipilimumab), a tyrosine kinase inhibitor (such as sunitinib, pazopanib, sorafenib, dasatinib, imatinib mesylate, lapatinib, or nilotinib), and a proteasome inhibitor (such as carfilzomib or bortezomib).

The subject in the present embodiment indicates any animal that can suffer from non-ischemic cardiomyopathy, and preferred examples thereof include an individual mammal, for example, a primate such as a human or a chimpanzee, a rodent such as a mouse, a rat, a guinea pig, or a hamster, an artiodactyl such as a cow, a goat, or sheep, a perissodactyl such as a horse, and an individual such as a rabbit, a dog, or a cat.

Further, in the treatment of diseases requiring prompt treatment for cardiomyopathy (particularly non-ischemic cardiomyopathy), it is desirable that mononuclear cells and dendritic cells after the culturing step or dendritic cells pulsed with α-GalCer are prepared in advance and cryopreserved, the pharmaceutical according to the present embodiment is promptly prepared from the cryopreserved cells after the onset, and the pharmaceutical is administered to the patient at an appropriate time. The effect of administration of the dendritic cells pulsed with α-GalCer tends to be exhibited 0 to 7 days after the administration, and the administration is particularly effective for treatment and rehabilitation of cardiomyopathy patients in an acute phase.

The pharmaceutical according to the present embodiment can be used for the purpose of preventing and treating diseases. In a case where the pharmaceutical is used for the purpose of prevention, the onset of cardiomyopathy can be prevented by administering the pharmaceutical according to the present embodiment to a subject who may suffer from a disease. For example, the onset of cardiomyopathy can be prevented by administering the pharmaceutical according to the present embodiment to a patient to whom a drug that can cause the above-described drug-induced cardiomyopathy has been administered or is intended to be administered. In a case where the pharmaceutical is used for the purpose of treatment, the progression of cardiomyopathy can be delayed, the progression of cardiomyopathy is stopped and the condition of a patient is improved, or the disease can be cured by administering the pharmaceutical according to the present embodiment to a subject who has been diagnosed with cardiomyopathy or whose symptoms have been confirmed.

The pharmaceutical according to the present embodiment contains an effective amount of dendritic cells pulsed with α-GalCer. The "effective amount" used in the present specification indicates an amount that is effective in preventing and/or treating a disease. Such an effective amount is appropriately adjusted depending on the type of disease, the severity of symptoms, and the patient and other medical factors.

In the preferred embodiment of the pharmaceutical according to the present embodiment, the effective amount of dendritic cells is in a range of 10⁶ to 10⁹ cells and preferably in a range of 10⁷ to 10⁹ cells per body surface area of 1 m² of an individual to which the pharmaceutical is administered.

The pharmaceutical according to the present embodiment is typically used in the form of a parenteral preparation such as an injection or an infusion. Examples of carriers that can be used for the parenteral preparations include aqueous carriers that are typically used for cell preparations such as physiological saline and isotonic solutions containing glucose, D-sorbitol, and the like.

The pharmaceutical according to the present embodiment may be a composition further comprising a pharmaceutically acceptable buffer, stabilizer, preservative, and other optional components. The pharmaceutically acceptable components are well known to those skilled in the art, and those skilled in the art can appropriately select components from among the components described in the Japanese Pharmacopoeia, Sixteenth Edition and other specifications within a range of normal implementation ability, according to the form of the preparation and then used.

In addition, the pharmaceutical according to the present embodiment may further contain a drug that may cause drug-induced cardiomyopathy (for example, a drug combination with an anthracycline anticancer agent). In this case, the complications of drug-induced cardiomyopathy can be prevented, desired treatments for cancer and the like can be continued, and the QOL of a patient can be improved.

The present embodiment also has an aspect of a method of preventing and/or treating non-ischemic cardiomyopathy, the method comprising administering the pharmaceutical to a subject in need thereof.

The subject to whom the pharmaceutical is administered is not limited to a patient suffering from non-ischemic cardiomyopathy, and may be a patient to whom a drug that may cause drug-induced cardiomyopathy has been administered or is intended to be administered.

The method of administering the pharmaceutical according to the present embodiment is not particularly limited, and in a case the pharmaceutical is in the form of a parenteral preparation, for example, the pharmaceutical is administered by intravascular administration (preferably intravenous administration), intraperitoneal administration, intestinal administration, subcutaneous administration, or the like. In one of the preferred embodiments, the pharmaceutical according to the present embodiment is administered to a living body by intravenous administration. Further, the pharmaceutical of the present invention may be used in combination with other pharmaceuticals depending on the disease to be prevented and/or treated. For example, the onset of drug-induced cardiomyopathy can be prevented by administering the pharmaceutical to a patient to whom a drug that can cause drug-induced cardiomyopathy has already been administered. The pharmaceutical may be administered to a patient simultaneously with the administration of a drug that may cause drug-induced cardiomyopathy (for example, an anthracycline anticancer agent) or a drug combination obtained by combining the pharmaceutical with a drug that may cause drug-induced cardiomyopathy may be administered.

A second embodiment of the present invention relates to a method of inhibiting a decrease in myocardial contractility, the method comprising administering the pharmaceutical containing dendritic cells obtained by a method comprising a step of culturing mononuclear cells in the presence of GM-CSF and IL-2 and a step of pulsing the cultured cells with α-galactosylceramide, to a subject in need thereof. The definition of each term in the present embodiment is the same as in the first embodiment.

The subject to whom the pharmaceutical is administered is not limited to a patient suffering from non-ischemic cardiomyopathy, and may be a patient suffering from a disease associated with a decrease in myocardial contractility. A decrease in myocardial contractility can cause cardiovascular diseases in peripheral blood vessels, kidneys, and the like and is likely to cause a decrease in QOL of a patient and a poor prognosis.

A third embodiment of the present invention relates to a method of preventing myocardial cell death, comprising administering the pharmaceutical comprising dendritic cells obtained by a method comprising a step of culturing mononuclear cells in the presence of GM-CSF and IL-2 and a step of pulsing the cultured cells with α-galactosylceramide, to a subject in need thereof. The definition of each term in the present embodiment is the same as in the first and second embodiments. Further, the present embodiment also has an aspect of a method of improving myocardial atrophy, the method comprising administering the pharmaceutical according to the first embodiment to a subject in need thereof. The use of an anthracycline-based anticancer agent is known to cause myocardial atrophy or myocardial cell death. According to the present embodiment, myocardial atrophy or myocardial cell death caused by an anthracycline-based anticancer agent can be inhibited.

A fourth embodiment of the present invention relates to a method of inhibiting progression of myocardial fibrosis (particularly, fibrosis of myocardial interstitium), comprising administering the pharmaceutical containing dendritic cells obtained by a method comprising a step of culturing mononuclear cells in the presence of GM-CSF and IL-2 and a step of pulsing the cultured cells with α-galactosylceramide, to a subject in need thereof. The definition of each term in the present embodiment is the same as in the first and second embodiments.

A fifth embodiment of the present invention relates to a method of inhibiting myocardial inflammation, the method comprising administering the pharmaceutical comprising dendritic cells obtained by a method comprising a step of culturing mononuclear cells in the presence of GM-CSF and IL-2 and a step of pulsing the cultured cells with α-galactosylceramide, to a subject in need thereof. The definition of each term in the present embodiment is the same as in the first and second embodiments.

The present invention will be described in more detail with reference to the following examples, but these examples are made to facilitate understanding of the present invention, and the technical scope of the present invention is not limited thereto.

### Examples

### 1. Doxorubicin-induced cardiomyopathy model mice (DOX mouse)

### (1) Preparation of DOX mice

Doxorubicin (6 mg/kg) was intravenously administered to male C57BL/6J mice aged 10 to 11 weeks from the tail vein once every two days. That is, in a case where the day when doxorubicin was initially administered was set to Day 0, doxorubicin was injected on Day 2 and Day 4 (total of 3 times of administration). At each time point of Day 0, 7, 14, 21, and 28, weight measurement and echocardiography were performed to evaluate the amounts of NKT cells and the expression levels of inflammatory cytokines in the myocardium.

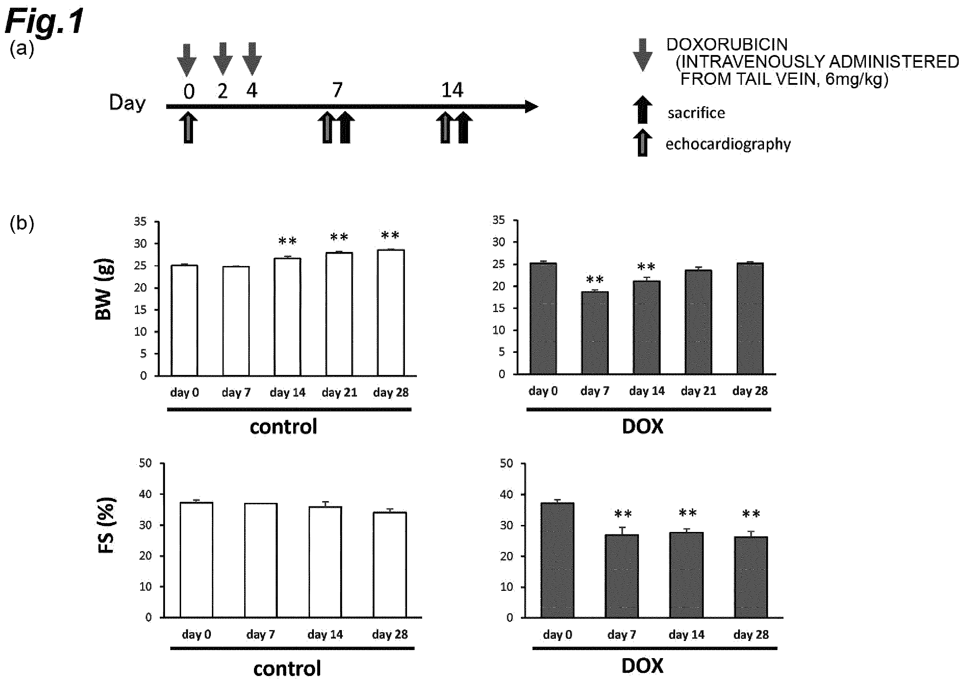

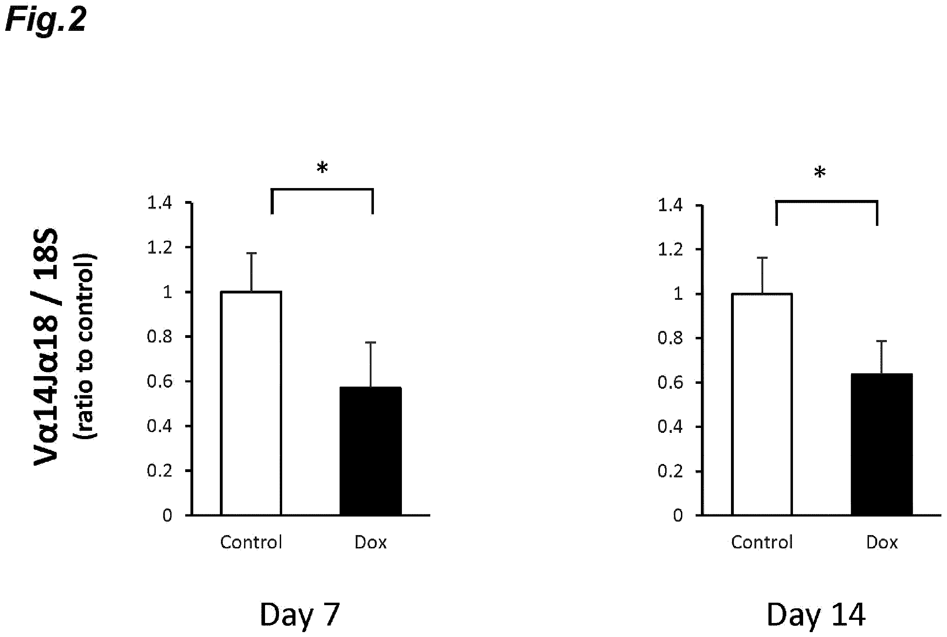

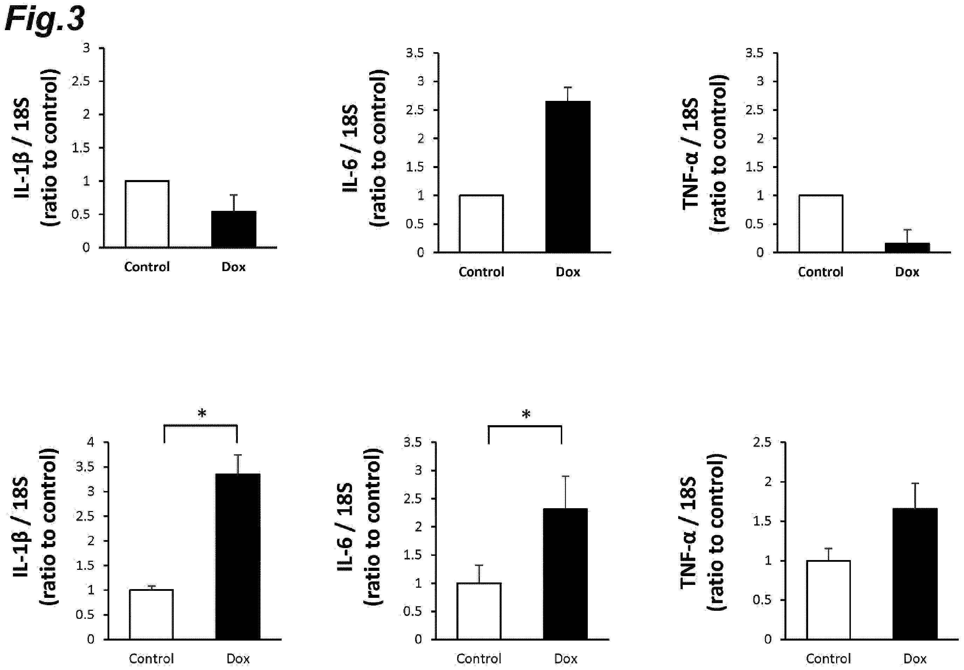

The results are shown in FIGS. 1 to 3. FIG. 1 is a graph showing changes over time in body weight (BW) and left ventricular fractional shortening rate (FS) at each time point of Days 0, 7, 14, 21, and 28. In FIG. 1, the asterisk (^{∗∗}) indicates that there is a significant difference (p < 0.01) as compared with the numerical value of Day 0. After administration of doxorubicin, the body weight that had been reduced after Day 7 tended to gradually increase, but it was found that the left ventricular fractional shortening rate was not improved. FIG. 2 is a graph showing the expression levels of Vα14Jα18 on Days 7 and 14. In FIG. 2, the asterisk (^{∗}) indicates that there is a significant difference (p < 0.05) as compared with the numerical value of Control. In a case where the doxorubicin non-administration group (Control) was set to 1, it was found that the expression level of Vα14Jα18 was decreased and the number of NKT cells was decreased in the doxorubicin administration group. FIG. 3 is a graph showing changes in inflammatory cytokines (IL-1β, IL-6, and TNF-α) on Days 7 and 14. In FIG. 3, the asterisk (^{∗}) indicates that there is a significant difference (p < 0.05) as compared with the numerical value of Control. On Day 7, the expression level of IL-6 in the doxorubicin administration group was significantly increased, and the expression levels of IL-1β and TNF-α were decreased. On Day 14, the expression levels of IL-1β, IL-6, and TNF-α were all significantly increased as compared with those of the doxorubicin non-administration group, and the expression levels of inflammatory cytokines were increased over a long period of time.

### 2. Effect of αGalCer on DOX mice

### (1) Cardiac function and inflammatory cytokines

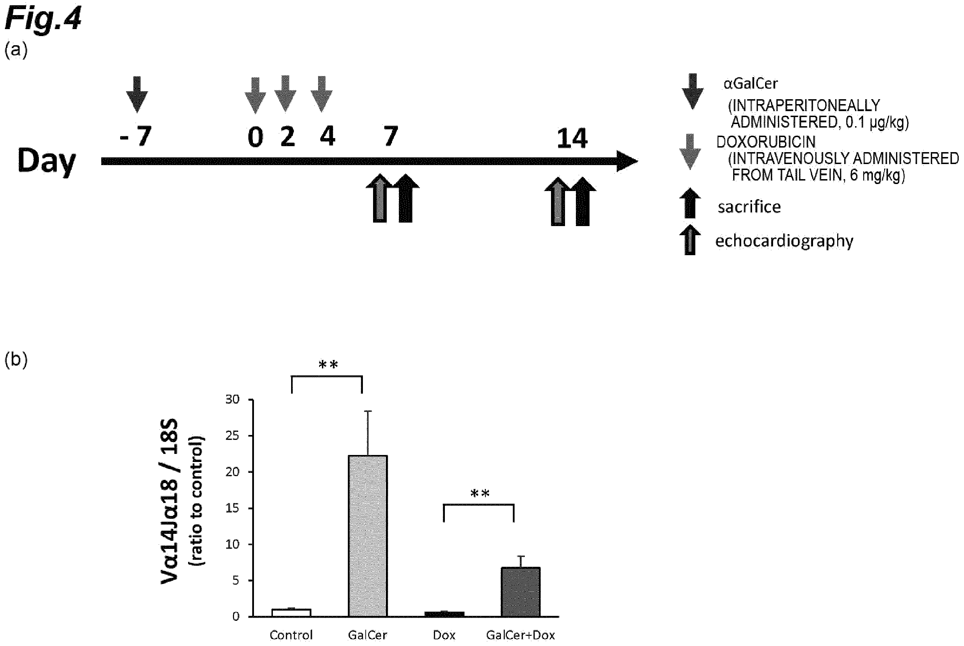

10-week-old male C57BL/6J mice were divided into four groups of a Control group, a GalCer group, a DOX group, and a GalCer + DOX group. Hereinafter, the administration method will be described by setting the day when doxorubicin was initially administered as Day 0 (FIG. 4 (a)).

PBS (phosphate buffered saline) was intraperitoneally administered (ip) and physiological saline was intravenously administered to the mice of the Control group on Day-7 (7 days before Day 0).

αGalCer (0.1 µg/g) was intraperitoneally administered (ip) to the mice of the GalCer group on Day-7, and physiological saline was intravenously administered thereto on Days 0, 2, and 4.

PBS was intraperitoneally administered (ip) to the mice of the DOX group on Day-7, and doxorubicin (6 mg/kg) was intravenously administered thereto on Days 0, 2, and 4.

αGalCer (0.1 µg/g) was intraperitoneally administered (ip) to the mice of the GalCer + DOX group on Day-7, and doxorubicin (6 mg/kg) was intravenously administered thereto on Days 0, 2, and 4.

The body weight, the blood pressure, the echocardiogram, the organ weight, the expression level of Vα14Jα18, the cardiac function, and inflammatory cytokines of each mouse at the time point of Day 14 were evaluated. Echocardiography was performed by anesthetizing mice with pentobarbital using an ultrasonic recording device EUB-8000 (trade name of Hitachi Medical Corporation). The left ventricular end-diastolic dimension and the left ventricular systolic end-systolic dimension were measured from the echo image, and the left ventricular fractional shortening rate serving as an index of the left ventricular contractility was calculated by an expression of "(left ventricular end-diastolic dimension - left ventricular systolic end-systolic dimension)/left ventricular end-diastolic dimension × 100".

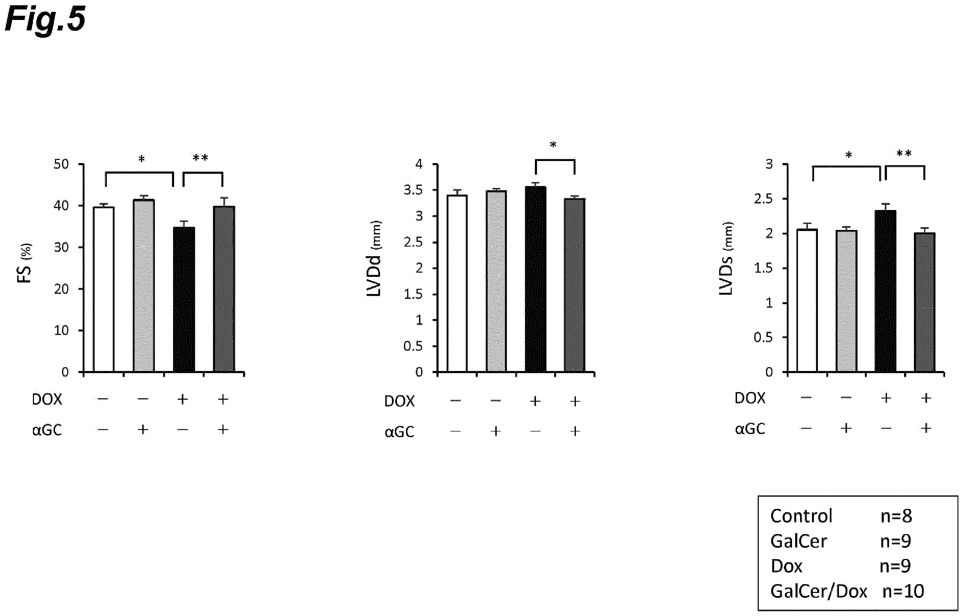

The results are shown in FIGS. 5 to 9. FIG. 4(b) is a graph comparing the expression levels of Vα14Jα18 in each group of mice. In FIG. 4 (b), the asterisk (^{∗∗}) indicates that there is a significant difference (p < 0.01) in the data between two groups. It was found that in a case where αGalCer was administered to mice with DOX cardiomyopathy, the expression level of Vα14Jα18 increased by approximately 10.6 times. FIG. 5 is a graph comparing the left ventricular fractional shortening rate (FS), the left ventricular end-diastolic dimension (LVDd), and the left ventricular end-systolic dimension (LVDs) of each group of mice. In FIG. 5, the asterisks (^{∗} and ^{∗∗}) indicate that there is a significant difference (p < 0.05 and p < 0.01 respectively) in the data between two groups. It was found that the left ventricular fractional shortening rate was small and the left ventricular end-systolic dimension was large in the mice of the DOX group, whereas the left ventricular fractional shortening rate and the left ventricular end-systolic dimension of the mice of the GalCer + DOX group improved to the same extent as the mice in the other groups. That is, the administration of GalCer results in an increase in NKT cells and improvement of a decrease in myocardial contractile function.

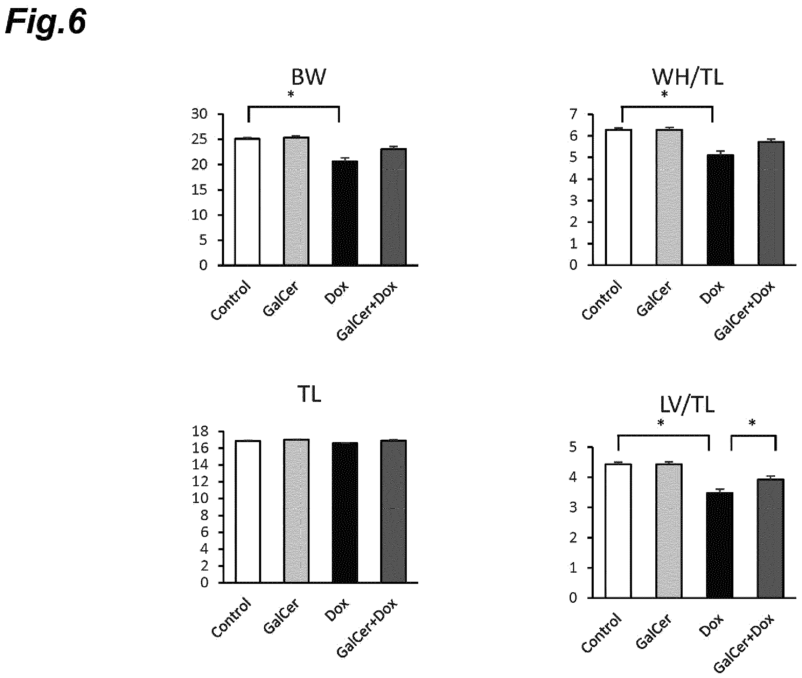

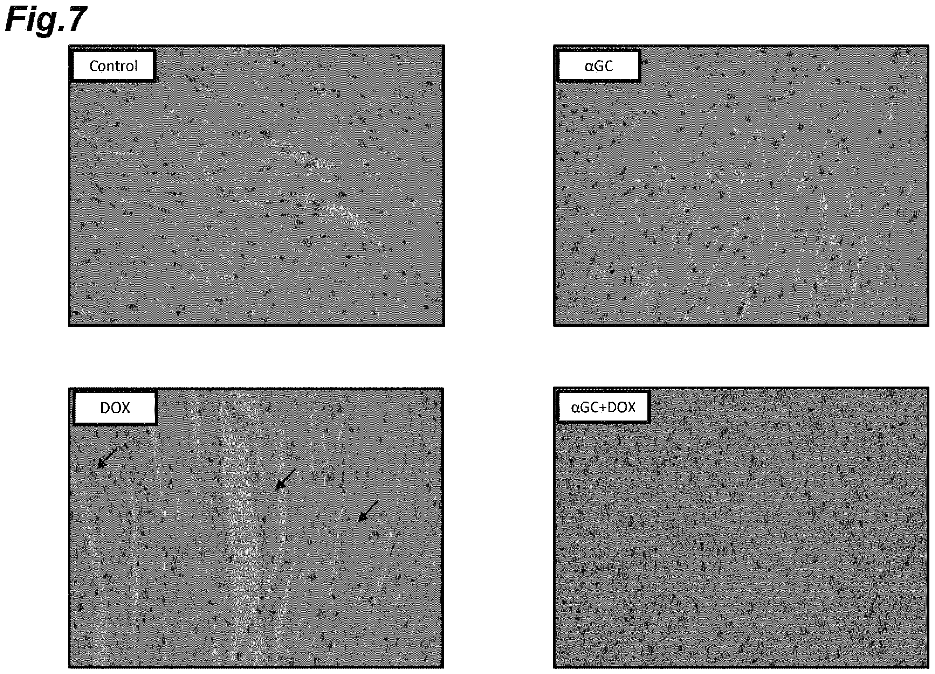

FIG. 6 is a graph comparing the body weight (BW), the true lumen (TL) in the echocardiogram, the ratio of the whole heart to the true lumen (WH/TL), and the ratio of the left ventricle to the true lumen (LV/TL) of each group of mice. In FIG. 6, the asterisk (^{∗}) indicates that there is a significant difference (p < 0.05) in the data between two groups. The values of WH/TL and LV/TL in the mice of the DOX group were significantly reduced as compared with the mice of the Control group. On the contrary, it was found that in the mice of the GalCer + DOX group, the values of WH/TL and LV/TL, which had been decreased in the mice of the DOX group, were improved. FIG. 7 is a photograph taken by TUNEL (terminal deoxynucleotidyl transferase dUTP nick end labeling)-staining myocardial cells collected from each group of mice. Apoptosis of myocardial cells was observed in the mice of the DOX group (indicated by arrows), but it was suggested that apoptosis was inhibited in the mice of the GalCer + DOX group. That is, apoptosis of myocardial cells was inhibited by administration of GalCer.

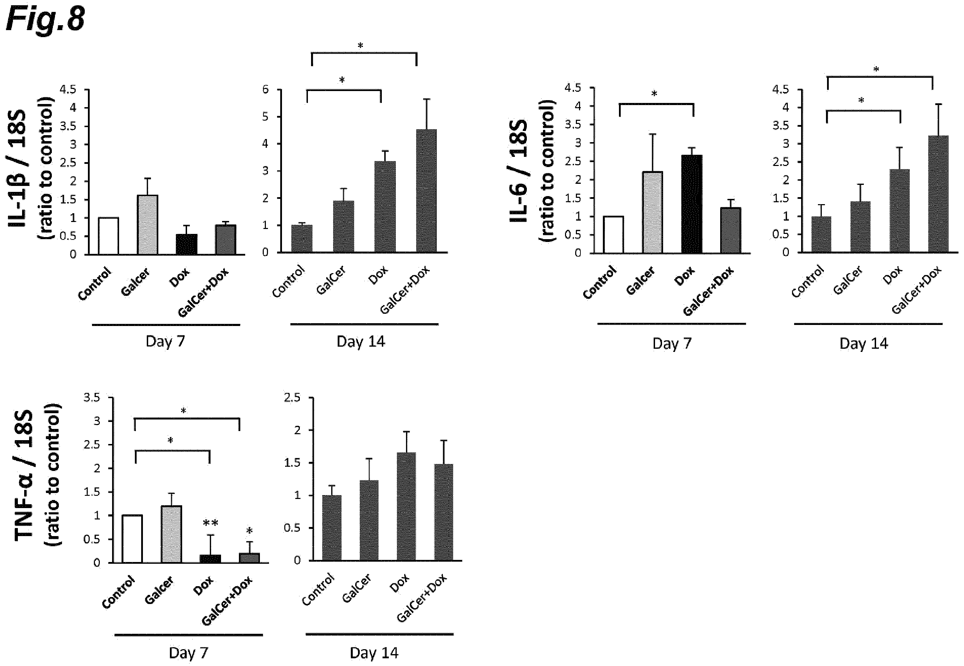

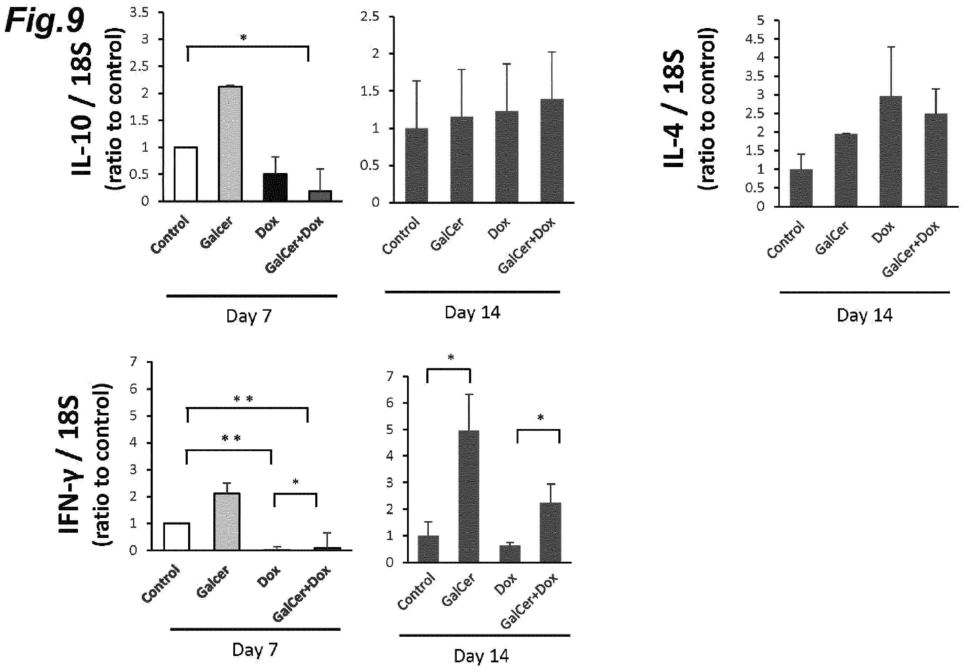

FIGS. 8 and 9 are graphs comparing the expression levels of inflammatory cytokines (IL-1β, IL-6, TNF-α, IL-10, IL-4, and IFN-γ) in each group of mice on Days 7 and 14. In FIGS. 8 and 9, the asterisks (^{∗} and ^{∗∗}) indicate that there is a significant difference (p < 0.05 and p < 0.01 respectively) in the data between two groups. That is, IFNγ was increased without decreasing inflammatory cytokines by administration of GalCer.

### 3. Effect of anti-IFN-γ antibody

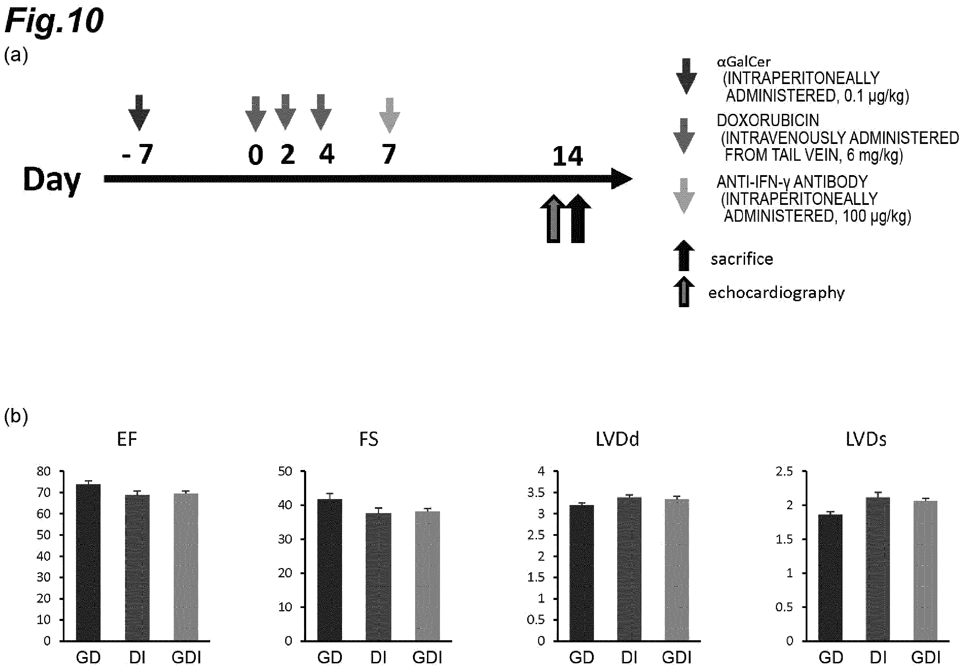

10-week-old male C57BL/6J mice were divided into three groups of a GD group, a DI group, and a GDI group. Hereinafter, the administration method will be described by setting the day when doxorubicin was initially administered as Day 0 (FIG. 10 (a)).

αGalCer (0.1 µg/g) was intraperitoneally administered (ip) to the mice of the GD group on Day-7, and doxorubicin (6 mg/kg) was intravenously administered thereto on Days 0, 2, and 4.

Doxorubicin (6 mg/kg) was intravenously administered to the mice of the DI group on Days 0, 2, and 4, and an anti-IFNγ antibody (100 µg) was intraperitoneally administered (ip) thereto on Day 7.

αGalCer (0.1 µg/g) was intraperitoneally administered (ip) to the mice of the GDI group on Day-7, doxorubicin (6 mg/kg) was intravenously administered thereto on Days 0, 2, and 4, and an anti-IFNγ antibody (100 µg) was intraperitoneally administered (ip) thereto on Day 7.

The echocardiogram, the organ weight, the cardiac function, and the inflammatory cytokines (IFN-γ) of each mouse at the time point of Day 14 were evaluated.

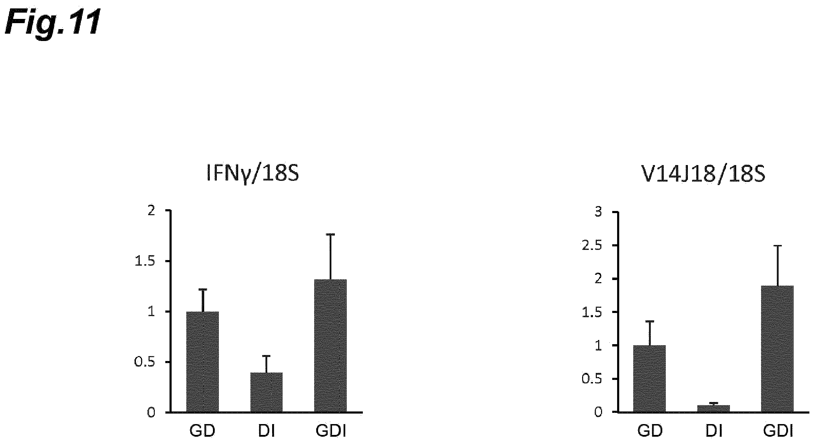

The results are shown in Table 1 and FIGS. 10 and 11. FIG. 10(b) is a graph comparing the ejection fraction (EF), the left ventricular fractional shortening rate (FS), the left ventricular end-diastolic dimension (LVDd), and the left ventricular end-systolic dimension (LVDs) of each group of mice. The heart rate (HR), the interventricular septum (IVS), and the posterior wall of the left ventricle (PW) are listed in Table 1. In the mice of the GD group, the ejection fraction and the left ventricular fractional shortening rate were increased, the left ventricular end-diastolic dimension and the left ventricular end-systolic dimension were decreased, and thus the improving effect on cardiomyopathy was obtained. On the contrary, in the mice of the GDI group, it was found that since these four indices were similar to those of the mice of the DI group, the cardioprotective effect of αGalCer was attenuated. FIG. 11 is a graph comparing the expression levels of IFNγ in myocardial cells of each group of mice. In the mice of the GD group, the expression level of IFNγ was increased as compared with the mice of the DI group, and this IFNγ increasing effect was attenuated due to the administration of the anti-IFNγ antibody. The amount of NKT cells was the highest in the mice of the GDI group.

**[Table 1]**

| | GD group | | DI group | | GDI group | |
|---|---|---|---|---|---|---|
| | Average | SD | Average | SD | Average | SD |
| HR | 511.1 | 16.0 | 520.4 | 8.4 | 527.3 | 10.2 |
| IVS | 0.86 | 0.05 | 0.79 | 0.06 | 0.77 | 0.02 |
| LVDd | 3.197 | 0.065 | 3.390 | 0.055 | 3.342 | 0.070 |
| LVDs | 1.858 | 0.050 | 2.117 | 0.073 | 2.064 | 0.038 |
| PW | 0.771 | 0.019 | 0.746 | 0.041 | 0.726 | 0.042 |
| EF | 73.80 | 1.84 | 68.69 | 1.89 | 69.59 | 1.01 |
| FS | 41.78 | 1.70 | 37.63 | 1.50 | 38.19 | 0.84 |

### 4. Heterologous tissue transplantation

Dendritic cells pulsed with α-GalCer were prepared by the method described in Non-Patent Literature 7. The specific procedures are described below.

### (1) Preparation of mononuclear cells

Apheresis extracellular fluid was collected from a healthy adult donor using a continuous blood component separator. 26.6 mL of the apheresis extracellular fluid was laminated on 20 mL of Ficoll-Paque PREMIUM (GE Healthcare Japan Corporation) in a 50 mL centrifugal tube and centrifuged at 400 × g and 20°C for 30 minutes to collect the mononuclear cell layer. The mononuclear cell layer was washed with the same amount of physiological saline and centrifuged at 400 × g and 20°C for 10 minutes. A suspension obtained by adding an albuminate-added AIM-V medium (a medium obtained by adding 1 volume of 4.4% KENKETU ALBUMINATE (trade name of Nihon Pharmaceutical Co., Ltd.) to 20 volumes of an AIM-V medium (manufactured by GIBCO Invitrogen Corporation)) to the sediment to have a liquid volume of 45 mL was centrifuge again. This operation was repeated once more, the obtained sediment was suspended in an autologous plasma-added albuminate-added AIM-V medium (a medium obtained by adding 1 volume of plasma collected from a heathy adult donor and 2 volumes of 4.4% KENKETU ALBUMINATE to 40 volumes of an AIM-V medium), and the liquid volume was adjusted such that the cell concentration was set to 2.7 × 10⁸ cells/mL or less to obtain a cell suspension.

13.8 mL of the cell suspension was dispensed into 25 mL freezing bags, 2 mL of an anticoagulant ACD-A solution (manufactured by Terumo Corporation) and 9.2 mL of a frost damage-protecting solution CP-1 (manufactured by Kyokuto Pharmaceutical Industrial Co., Ltd.) were added to each bag and then cryopreserved at -80°C.

### (2) Preparation of dendritic cells pulsed with α-GalCer

The mononuclear cells cryopreserved in the process (1) were thawed at 37°C, washed with 4.4% KENKETU ALBUMINATE whose volume was two times the volume of the mononuclear cells by adding 4.4% KENTETU ALBUMINATE, and then centrifuged at 400 × g and 20°C for 5 minutes. 45 mL of albuminate-added physiological saline was added to the sediment, and the cells were washed again and centrifuged to obtain a sediment of mononuclear cells.

1 × 10⁸ mononuclear cells were seeded in a 225 cm² flask and cultured at a temperature of 37°C and a CO₂ concentration of 5.0% for 7 days using 50 mL of an autologous plasma-added albuminate-added AIM-V medium prepared such that human IL-2 (manufactured by Shionogi & Co., Ltd.) was added at a final concentration of 100 JRU/mL and human GM-CSF (manufactured by North China Pharmaceutical Group Corporation-GeneTech) was added at a final concentration of 800 U/mL. 50 mL of an autologous plasma-added albuminate-added AIM-V medium was further added on the 3rd and 6th days of the culture, and human IL-2 was added at a final concentration of 100 JRU/mL and human GM-CSF was added at a final concentration of 800 U/mL. α-GalCer (manufactured by Funakoshi Co., Ltd.) was added at a final concentration of 100 ng/mL on the 6th day of the culture.

After completion of the culture, the cells were collected from the flask by a cell scraper and pipetting, filtered through a cell strainer, and washed with 45 mL of albumin-added physiological saline (obtained by adding 1 volume of 25% KENKETU ALBUMIN (trade name of The Chemo-Sero-Therapeutic Research Institute) to 10 volumes of physiological saline, and centrifuged at 400 × g and 20°C for 5 minutes. The sediment was washed three more times in the same manner as described above and then suspended in 10 mL of albumin-added physiological saline. The total amount of the cell suspension was collected with a syringe and added to a 50 mL physiological saline bottle. The resultant was used as α-GalCer-added dendritic cells (α-GalCer/DC) in the following examples.

### (3) Preparation of dendritic cells that had not been pulsed with α-GalCer and leukocytes

α-GalCer-free dendritic cells (DC) and α-GalCer-free leukocytes (Leukocyte) were prepared in the same manner as described above except that α-GalCer was not added.

### (4) Changes in NKT cells due to heterologous tissue transplantation

Each suspension (3 × 10⁶ cells/PBS 50 µL) of α-GalCer/DC, DC, and Leukocyte obtained in the above-described manner was prepared and injected into the tail veins of 5-week-old male Balb/c mice. As a Control, PBS (50 µL) was administered to Balb/c mice. On the 4th day after the administration, myocardial cells and the serum of each group of mice were collected, and the mRNA expression levels of Vα14Jα18 and inflammatory cytokines (IFN-γ, IL-4, and IL-10) were measured by the RT-PCR method.

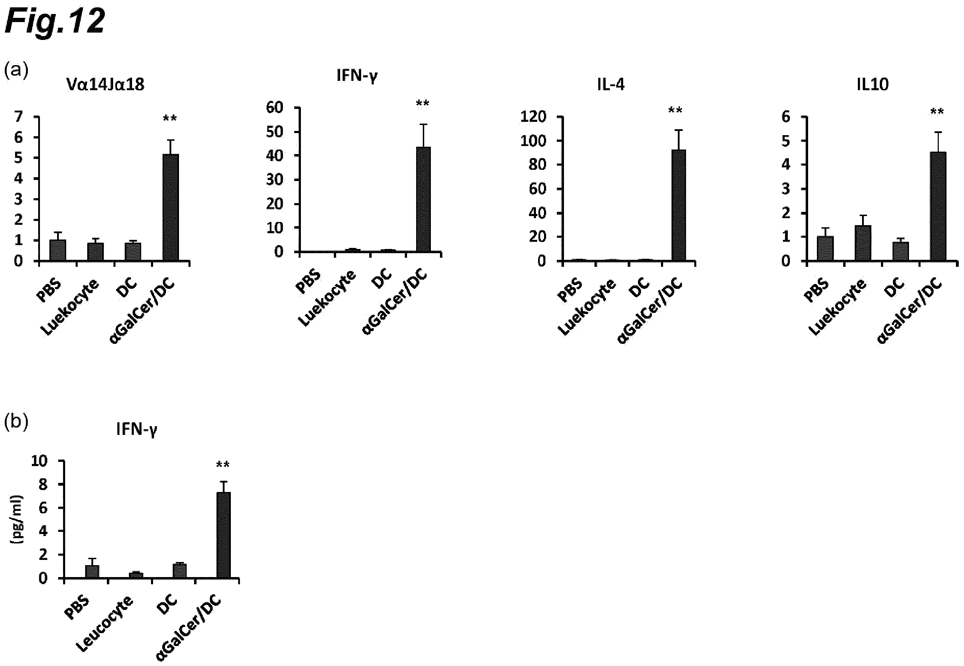

The results are shown in FIG.12. Each mRNA expression level is shown as a relative value with respect to the mRNA expression level in PBS group mice. In FIG. 12, the asterisk (^{∗∗}) indicates that there is a significant difference (p < 0.01) in the data between two groups. It was found that the mRNA expression levels of the mice in the DC group and the mice of the Leukocyte group were similar to the mRNA expression levels of the mice in the PBS group, and the mRNA expression levels did not change even in a case where dendritic cells or leukocytes derived from a human were simply administered. On the contrary, the mRNA expression levels of all the mice in the α-GalCer/DC group were significantly increased, and the mRNA expression levels of IFN-γ in the serum were also increased.

### (5) Sustainability of effect of α-GalCer/DC

The suspension of α-GalCer/DC (3 × 10⁶ cells/50 µL of PBS) obtained in the above-described manner was prepared and injected into the tail veins of 5-week-old male Balb/c mice. Myocardial cells were collected from the mice and the mRNAs expression levels of Vα14Jα18, IFN-γ, IL-4, and IL-10 were measured at the time points of the injection day (Day 0) and Days 1, 4, 7, 14, and 28.

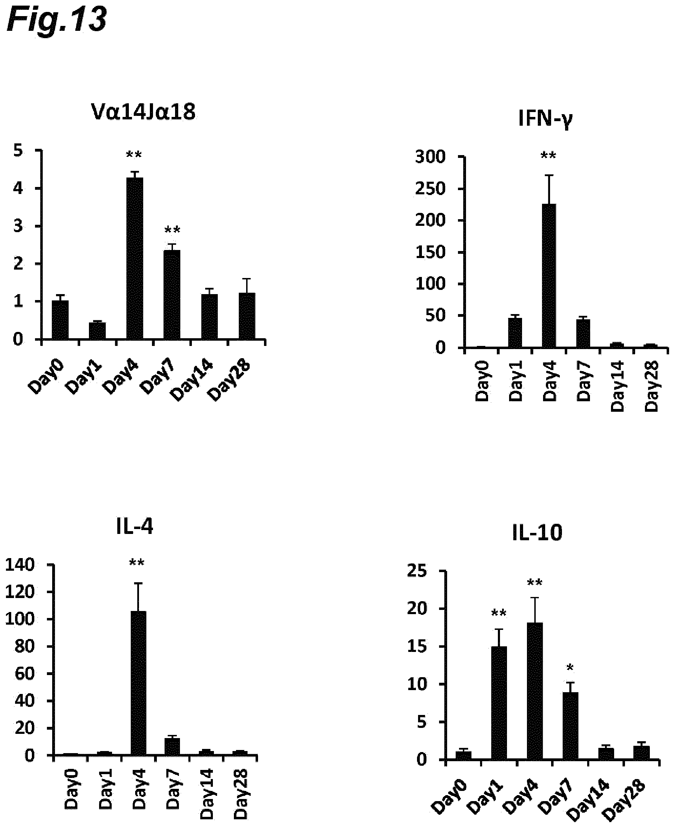

The results are shown in FIG. 13. In FIG. 13, the asterisks (^{∗} and ^{∗∗}) indicate that there is a significant difference (p < 0.05 and p < 0.01, respectively) with respect to the expression level on Day 0. It was confirmed that in a case where the suspension of α-GalCer/DC was administered once, the activation of NKT cells was highest on the 4th day and maintained until the 7th day. In addition, an increase in the expression level of IFN-γ was maintained until 28 days after the administration.

### 5. Administration to dilated cardiomyopathy model mouse

### (1) Preparation of dilated cardiomyopathy model mouse

ΔK210 knock-in mice (DCM mice) with Balb/c mice as the background were prepared as a model of troponin T mutant dilated cardiomyopathy with reference to Non-Patent Literatures 14 and 15. The prepared DCM mice exhibited severe left ventricular dilatation and decreased left ventricular ejection fraction (LVEF) at 5 weeks of age, and all the mice died of heart failure at approximately 9 weeks of age in a case where pimobendan was not administered.

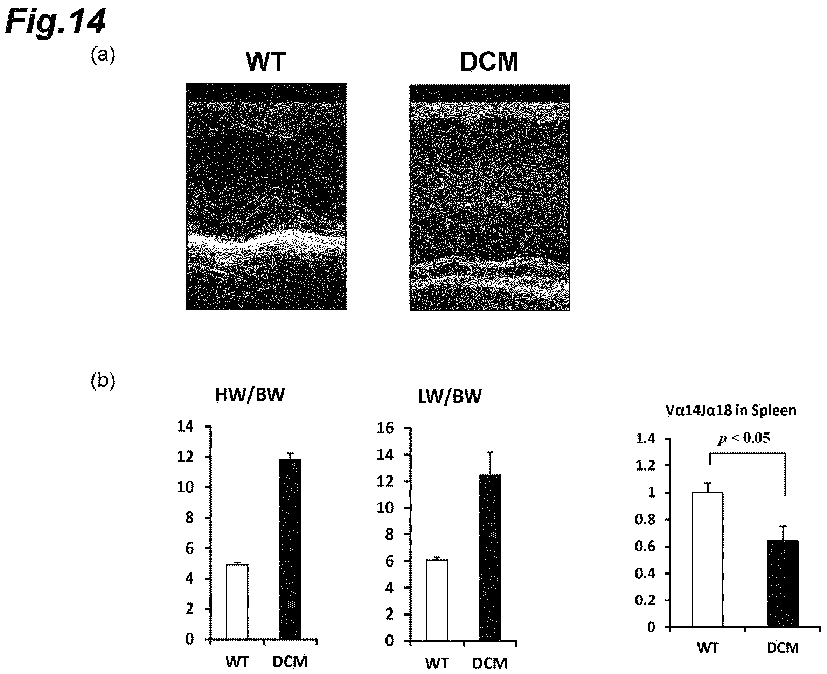

FIG. 14(a) is a photograph showing a cross section of a myocardial tissue of a DCM mouse and a wild-type Balb/c mouse (WT mouse). FIG. 14(b) is a graph comparing the heart weight HW/BW and the lung weight LW/BW of both mice and a graph comparing the expression levels of Vα14Jα18 in the spleen.

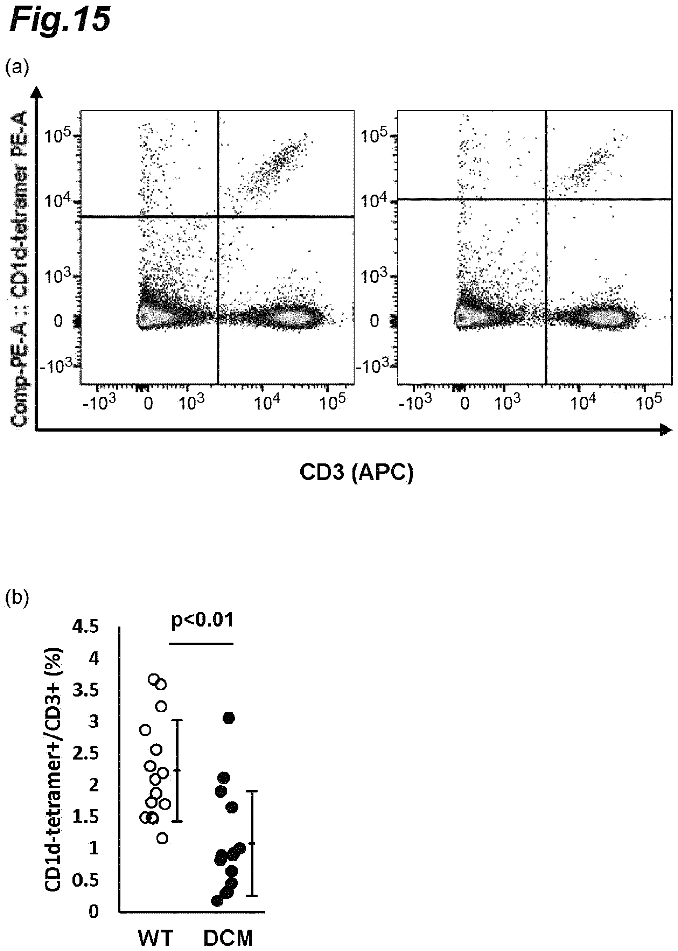

In addition, spleen cells were collected from DCM mice and WT mice, and the cells were separated from each other using a PE-labeled CD1d tetramer antibody and an anti-CD3 antibody. The obtained cytogram is shown in FIG. 15. The graph shows the proportion of CD3⁺CD1d tetramer⁺ cells in CD3⁺ cells. It was found that the heart weight and the lung weight of the DCM mice were larger than those of the WT mice, and the amount of NKT cells of the DCM mice was smaller than that of the WT mice.

### (2) Administration of α-GalCer/DC to DCM mouse (evaluation at distant time)

The suspension of α-GalCer/DC (3 × 10⁶ cells/PBS 50 µL) obtained in the above-described manner or PBS (50 µL) was injected into the tail veins of 5-week-old WT or DCM mice. Thereafter, the state of the mice was observed up to 9 weeks of age (4 weeks after the administration), and the cardiac function was evaluated based on the echocardiogram of the survived mice, the tissues were analyzed, and the changes in the expression levels of the cytokines were analyzed.

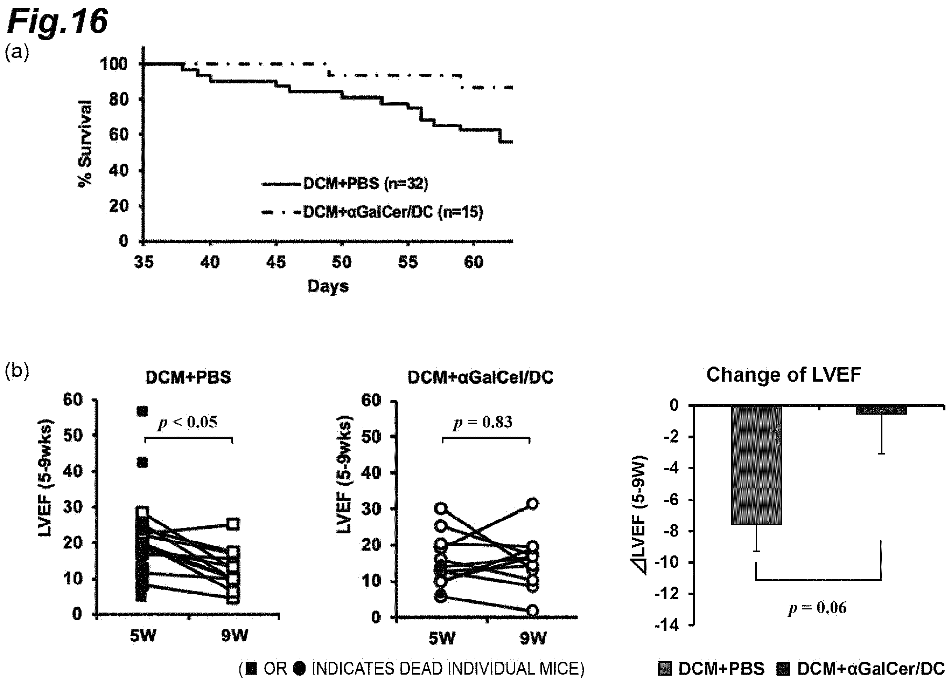

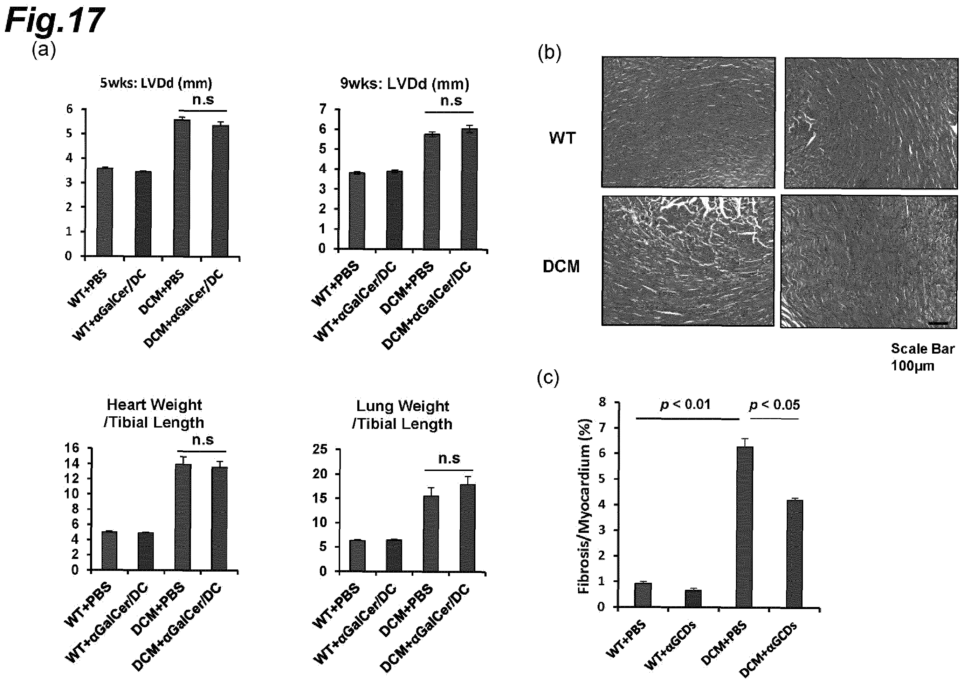

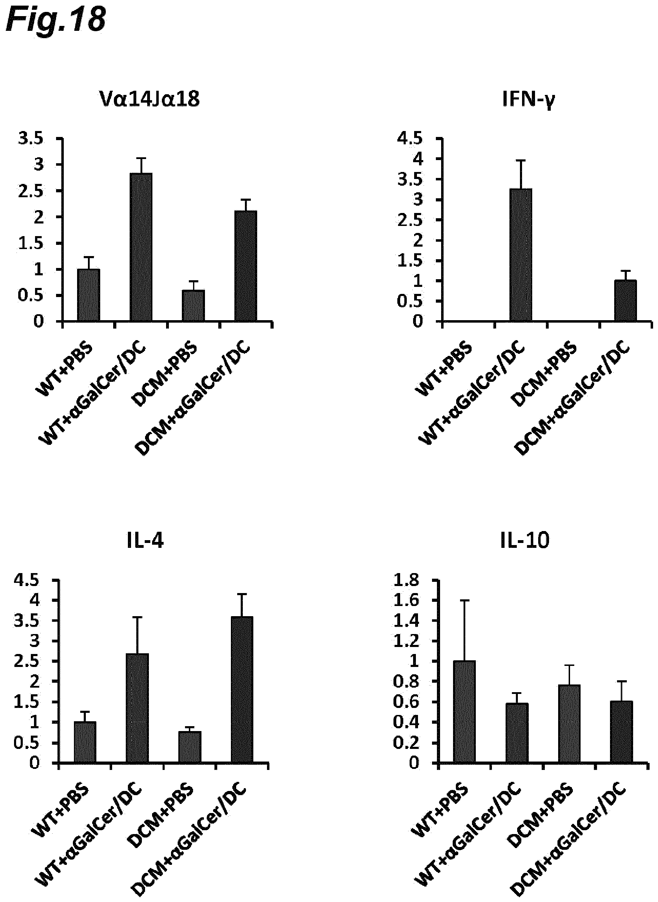

The results are shown in FIGS. 16 to 18. FIG. 16(a) is a graph showing the survival rate of DCM mice over time. In the comparison between the two groups, a difference in survival rate began to be seen early after the administration, and a statistically significant difference (p < 0.05) was obtained at 9 weeks of age. FIG. 16(b) is a graph showing changes in left ventricular ejection fraction (LVEF) of 5-week-old (at the time of administration) and 9-week-old DCM mice. LVEF was significantly decreased in the PBS administration group, whereas no significant decrease was observed in the α-GalCer/DC administration group. In comparison of the amount of change in LVEF over time between the two groups of mice, it was found that there was a tendency to inhibit the decrease in LVEF. FIG. 17(a) is a graph showing the left ventricular end-diastolic dimension (LVDd) of 5-week-old and 9-week-old mice and the heart weight (HW/TL (Tibial length)) and the lung weight (LW/TL) of 9-week-old mice in four groups. Significant changes in the left ventricular end-diastolic dimension, the heart weight, and the lung weight were not found due to the administration of αGalCer/DC. FIG. 17(b) is a photograph showing a cross section of myocardial cells. FIG. 17(c) is a graph showing the proportion of fibrotic cells of the myocardial interstitium in the four groups. The proportion of the fibrotic cells was significantly smaller in the DCM mice to which α-GalCer/DC had been administered, as compared with that in the DCM mice (DCM + PBS group) to which PBS had been administered. That is, fibrosis of the myocardial interstitium was inhibited due to the administration of α-GalCer/DC.FIG. 18 is a graph comparing the expression levels of Vα14Jα18 and cytokines (IFN-γ, IL-4, and IL-10) in the four groups. In both the WT and DCM mice, the expression levels of Vα14Jα18, IFN-γ, and IL-4 were increased and the expression levels of IL-10 were decreased due to the administration of α-GalCer/DC.These changes were also observed 4 weeks after the administration of α-GalCer/DC.

### (3) Administration of α-GalCer/DC to DCM mouse (early evaluation)

The suspension of α-GalCer/DC (3 × 10⁶ cells/PBS 50 µL) obtained in the above-described manner or PBS (50 µL) was injected into the tail veins of 5-week-old WT or DCM mice. On the 4th day after the administration, the cardiac function was evaluated based on the echocardiogram of each group of mice and the changes in the expression levels of cytokines were analyzed.

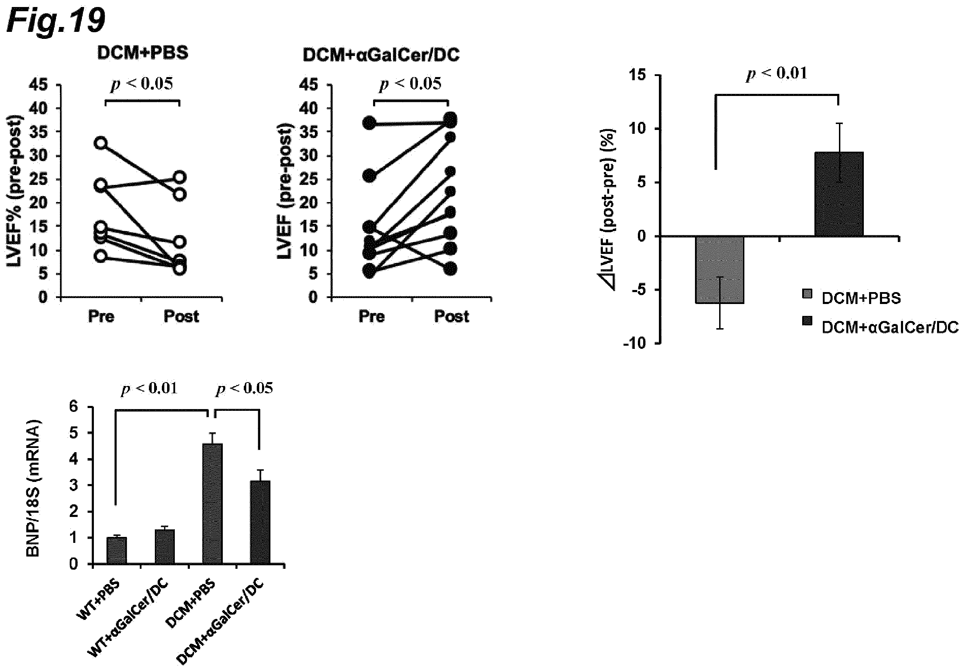

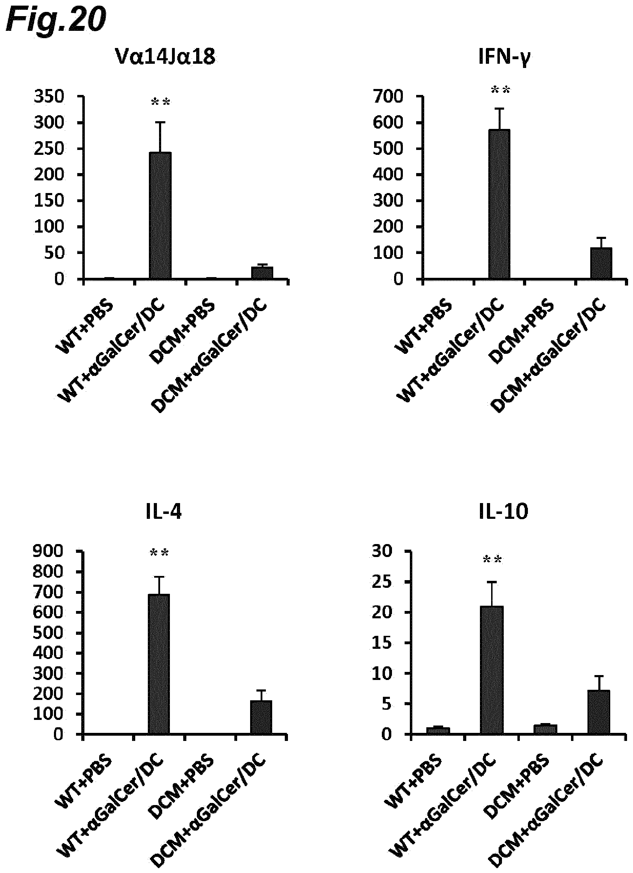

The results are shown in FIGS. 19 and 20. FIG. 19 is a graph showing changes in left ventricular ejection fraction (LVEF) before and after administration of α-GalCer/DC or PBS in DCM mice. It was found that LVEF was significantly decreased in the PBS administration group, whereas LVEF was significantly increased in the α-GalCer/DC administration group. In addition, the mRNA expression levels of brain natriuretic peptide (BNP) were significantly reduced due to the administration of α-GalCer/DC.FIG. 20 is a graph showing the expression levels of inflammatory cytokines in the four groups. The graph of FIG. 20 was recorded as relative values with respect to the WT + PBS group (the group in which PBS had been administered to WT mice). In FIG. 20, the asterisk (^{∗∗}) indicates that there is a significant difference (p < 0.01) with respect to the data of the WT + PBS group. It was found that the expression levels of various cytokines (IFN-γ, IL-4, and IL-10) were changed due to the administration of α-GalCer/DC.

### (4) Changes in expression level of cytokines in blood

In the 4 groups in the process (3) described above, the concentrations of cytokines (IFN-γ, IL-4, and IL-10) in blood at the time point of Day 4 were measured. Further, α-GalCer/DC was injected into the tail veins of Balb/c mice for comparison, and the concentrations of IFN-γ in blood at each time point of Days 1, 4, 7, 14, and 28 were measured.

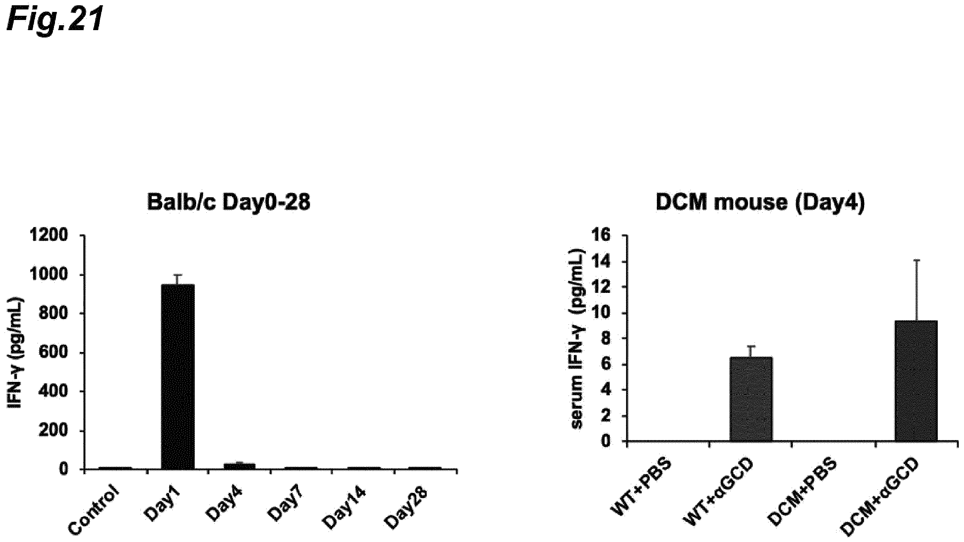

The results are shown in FIG. 21. It was found that the concentrations of IFN-γ in blood were significantly increased during the period of Days 1 to 4 in a case of the administration to the Balb/c mice. In addition, the concentrations of IFN-γ in blood were significantly increased even in the DCM mice. On the contrary, the concentrations of IL-4 and IL-10 in blood were below the lower limit of detection.

### 6. Acute effect of α-GalCer/DC

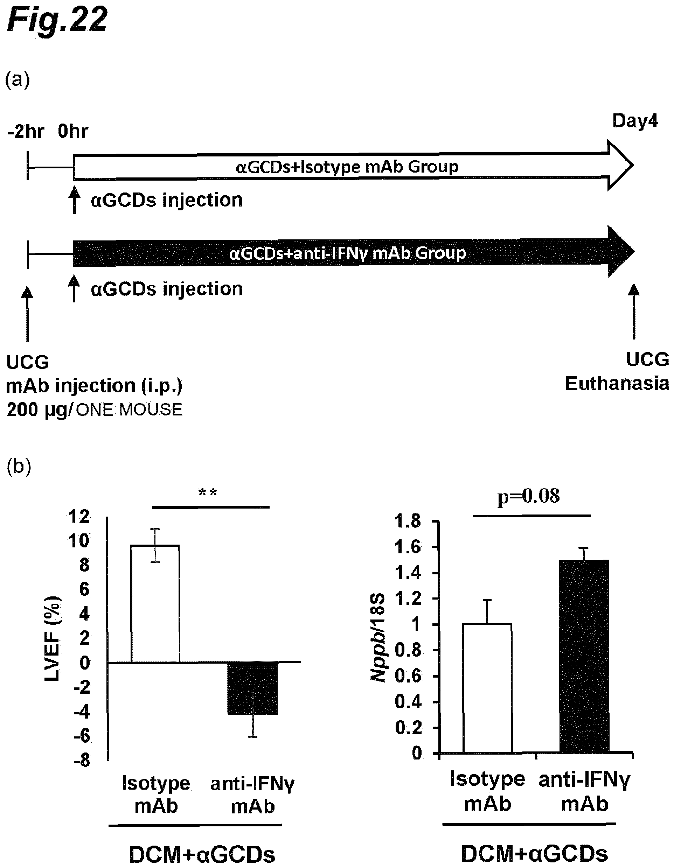

After the echocardiogram of the DCM mice was measured, an anti-mouse IFN-γ antibody or an isotype antibody (200 µg/one mouse) was intraperitoneally administered (FIG. 22 (a)). Two hours after the administration of the antibody, the suspension of α-GalCer/DC (3 × 10⁶ cells/50 µL of PBS) was injected into the tail veins (Day 0), and the echocardiogram was measured on Day 4.

The results are shown in FIG. 22 (b). The left ventricular ejection fraction (LVEF) increased in the isotype antibody administration group, whereas the LVEF was decreased in the anti-IFN-γ antibody administration group, and accordingly, a significant difference was observed between the two groups. Further, the expression levels of the natriuretic peptide B (Nppb) were also significantly increased in the anti-IFN-γ antibody administration group. In this manner, it was found that the acute contractile function improving effect of α-GalCer/DC was mediated by IFN-γ.

### 7. Phosphorylation induction of PLN in myocardium

### (1) DCM mouse

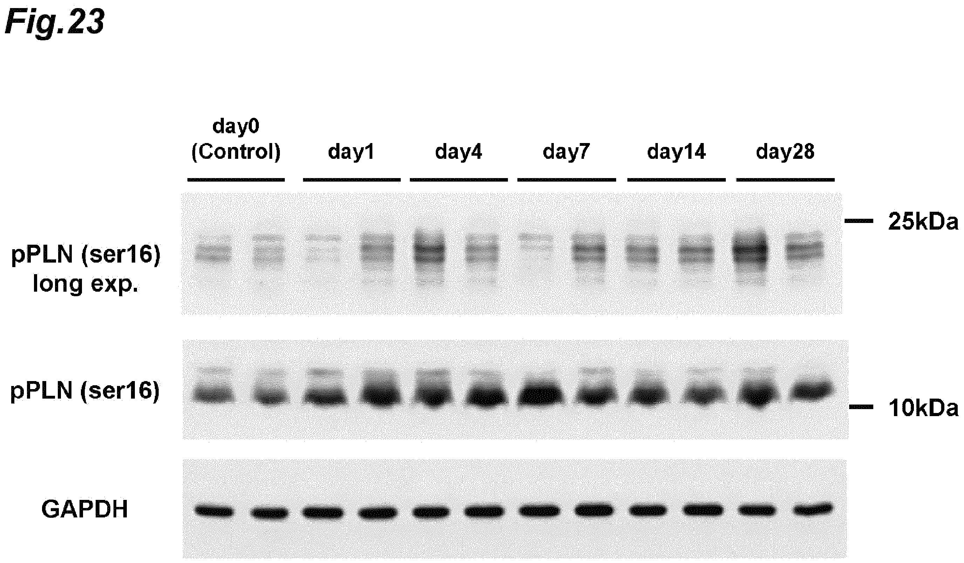

The suspension of α-GalCer/DC (3 × 10⁶ cells/50 µL of PBS) was injected into the tail veins of the Balb/c mice (Day 0), the myocardial cells collected from the DCM mice were crushed at each time point of Day 0, 1, 4, 7, 14 and 28, and the expression levels of PLN, phosphorylated PLN (p-PLN, PLN in which Ser16 was phosphorylated), and GAPDH (glyceraldehyde 3-phosphate dehydrogenase) were evaluated by the SDS-PAGE method. As shown in FIG. 23, the concentration of pPLN began to increase slightly after the administration of α-GalCer/DC and reached the highest value on Days 1 to 7.

### (2) Cultured myocardial cells

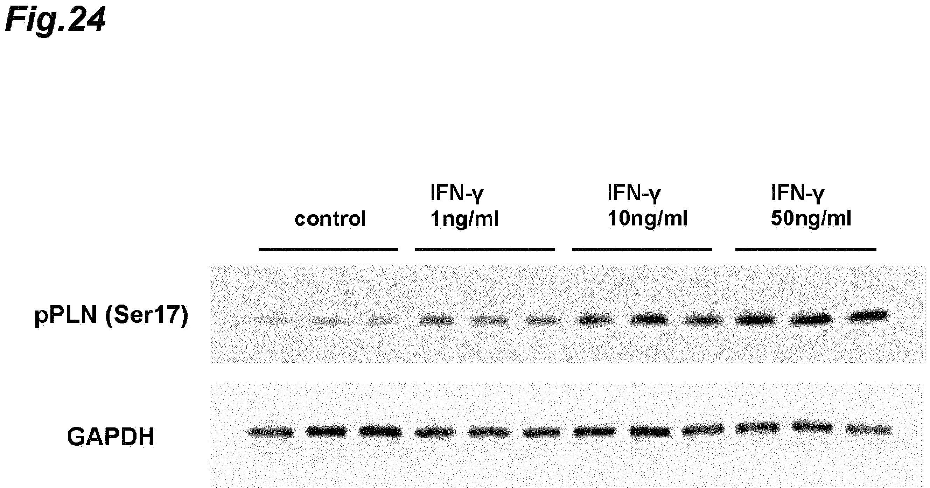

The cultured myocardial cells were stimulated by adding IFN-γ solutions with three different concentrations (1, 10, and 50 ng/mL), and changes in concentration of pPLN were evaluated by the SDS-PAGE method. As shown in FIG. 24, it was found that PLN was phosphorylated when cultured myocardial cells were stimulated with IFN-γ.

### (3) Cultured myocardial cells

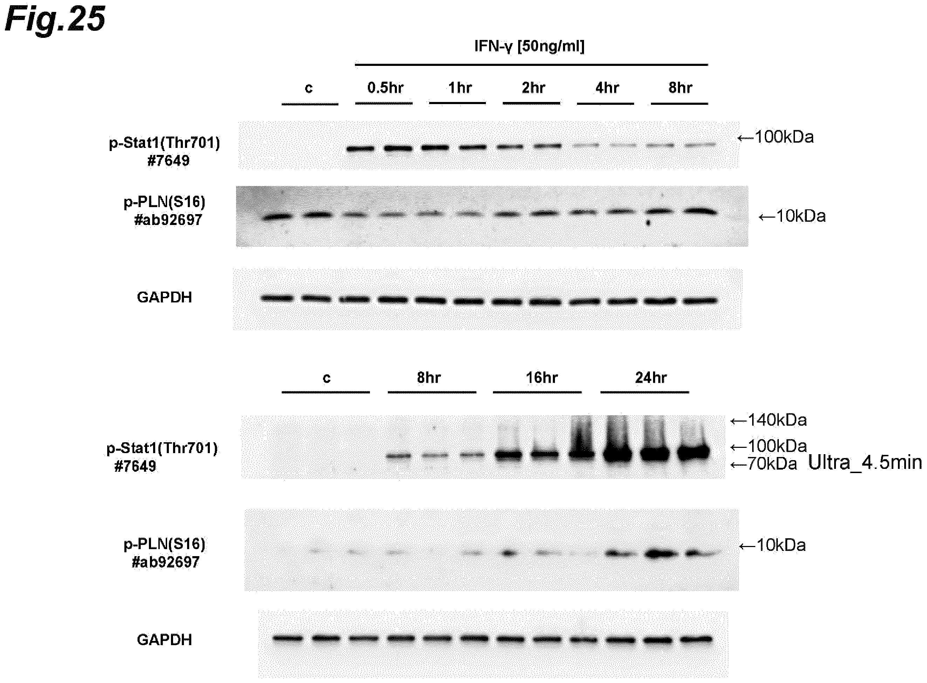

The cultured myocardial cells were stimulated by adding 50 ng/mL of an IFN-γ solution, and a change in concentration of pPLN over time was evaluated by the SDS-PAGE method. As shown in FIG. 25, when cultured myocardial cells were stimulated with IFN-γ, p-Stat1 (phosphorylated Stat1, Stat1 in which The701 was phosphorylated) was increased immediately after the stimulation, and pPLN was phosphorylated with a slight delay.

### (4) GO analysis of microarray

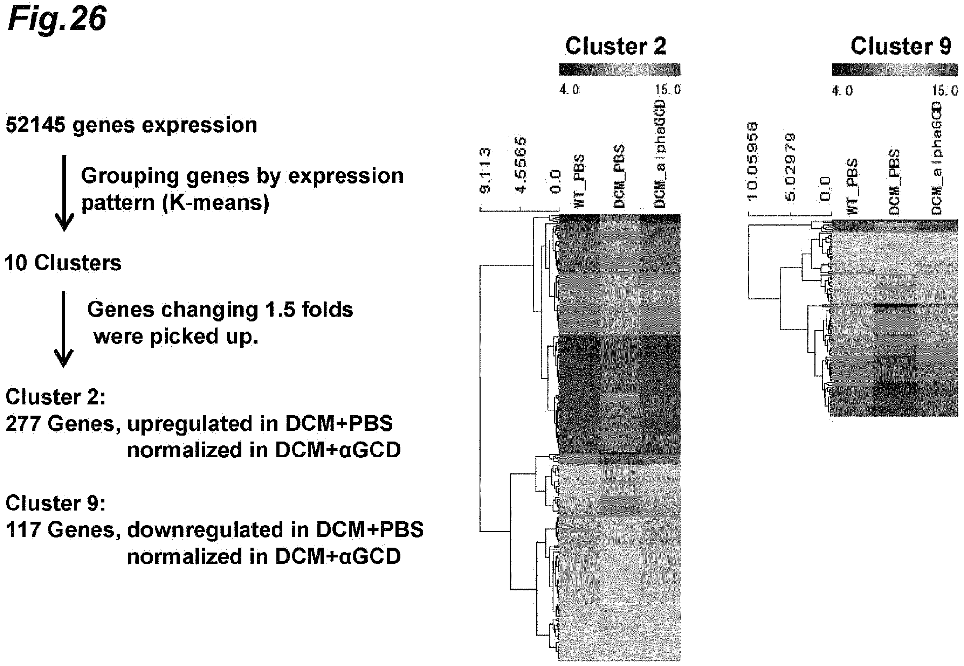

Microarray analysis was performed on each of the WT + PBS group, the DCM + PBS group, and the DCM + α-GalCer/DC group described above, and genes whose expression was increased or decreased were extracted. As shown in FIG. 26, 277 genes (cluster 2) whose expression was increased in the DCM + PBS group and whose expression was not changed in the DCM + α-GalCer/DC group were identified. In addition, 117 genes (cluster 9) whose expression was decreased in the DCM + PBS group and whose expression was not changed in the DCM + α-GalCer/DC group were identified. The decreased expression of angiogenesis-related genes was increased, the attenuated immune response-related genes were increased, the decreased expression of fatty acid metabolism-related genes was normalized, and the gene expression representing increased fibrosis was normalized in the myocardium due to the administration of α-GalCer/DC to the DCM mice.

Further GO analysis was performed on clusters 2 and 9 identified from genes obtained from the hearts of the DCM mice 4 days after the administration of α-GalCer/DC.The obtained results are listed in Tables 2 and 3. In particular, the cellular response to transforming growth factor β (TGFβ) stimulation was paid attention in the cluster 2, and the angiogenesis was paid attention in the cluster 9.

**[Table 2]**

| Cluster 2: GO Term | Count | % | P value |
|---|---|---|---|
| fat cell differentiation | 4 | 9.1 | < 0.001 |
| cellular response to fibroblast growth factor stimulus | 3 | 6.8 | 0.002 |
| positive regulation transcription from RNA polymerase II promoter | 8 | 18.2 | 0.007 |
| cellular response to transforming growth factor beta stimulus | 3 | 6.8 | 0.008 |
| response to mechanical stimulus | 3 | 6.8 | 0.009 |
| positive regulation of endothelial cell proliferation | 3 | 6.8 | 0.010 |
| positive regulation of osteoblast differentiation | 3 | 6.8 | 0.010 |
| endothelial cell chemotaxis | 2 | 4.5 | 0.022 |

**[Table 3]**

| Cluster 9: GO Term | Count | % | P value |
|---|---|---|---|
| morphogenesis of an epithelium | 4 | 3.7 | < 0.001 |
| vasculogenesis | 5 | 4.6 | < 0.001 |
| fatty acid metabolic process | 6 | 5.6 | 0.001 |
| positive regulation of phagocytosis | 4 | 3.7 | 0.002 |
| cellular response to tumor necrosis factor | 5 | 4.6 | 0.002 |
| response to lipopolysaccharide | 6 | 5.6 | 0.003 |
| innate immune response | 8 | 7.4 | 0.004 |
| inflammatory response | 7 | 6.5 | 0.008 |

### 8. Comparison of expression levels of genes involved in fibrosis and angiogenesis

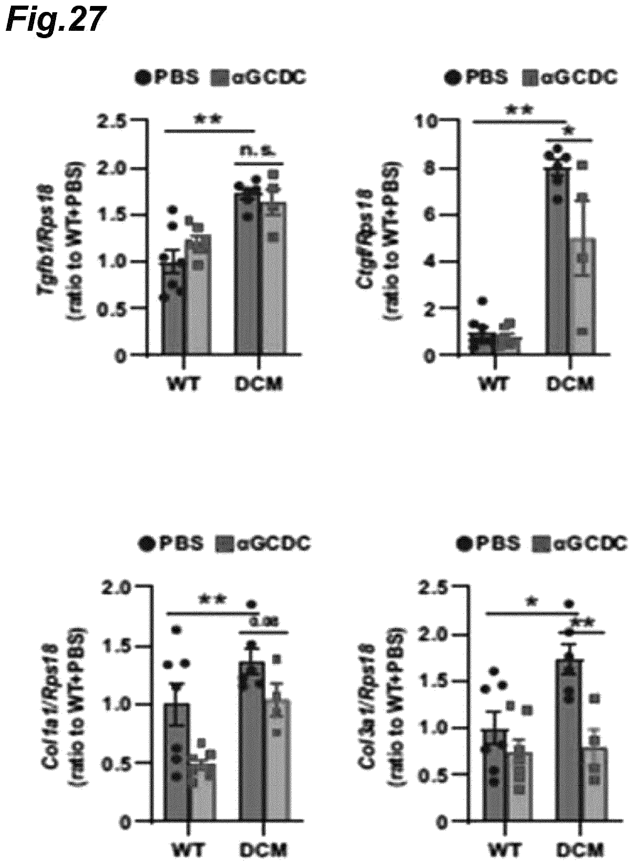

The expression levels of genes involved in fibrosis and angiogenesis were evaluated 4 days after the administration of PBS or α-GalCer/DC (number of administered cells: 3 × 10⁶) to wild mice (WT) or DCM mice (DCM). The results are shown in FIGS. 27 to 29.

FIG. 27 is a graph showing the expression levels of mRNA in the myocardium of wild mice (WT) or DCM mice (DCM) (n = 4 to 6). Tgfb is classified into the cluster 2 by GO analysis of microarray. Rps18 was used as an intrinsic control. In the wild mice (WT) and the DCM mice (DCM), the expression levels of Tgfb did not change significantly regardless of the administration of α-GalCer/DC. However, the mRNA expression levels of the genes (Ctgf, Col1a, and Col3a) involved in fibrosis on the downstream side thereof were significantly inhibited by the administration of α-GalCer/DC.

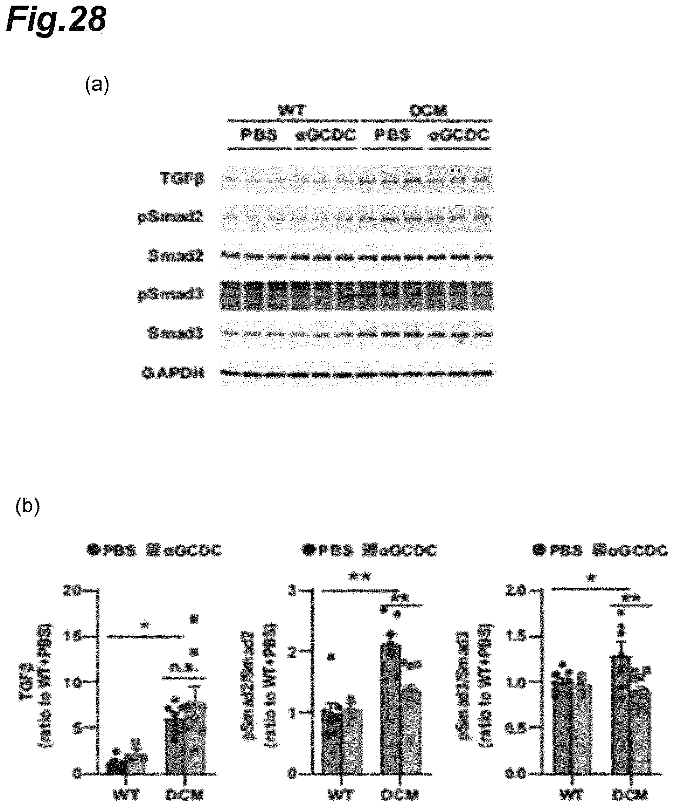

FIG. 28 is a western blot showing the expression levels of proteins (TGFβ, pSmad2, Smad2, pSmad3, and Smad3) in the myocardium of wild mice (WT) or DCM mice (DCM) (n = 3 to 8). GAPDH was used as an intrinsic control. FIG. 28 (b) is a graph quantifying the results of FIG. 28 (a). pSmad2 and pSmad3 each indicate phosphorylated Smad2 and phosphorylated Smad3. At the protein levels, the expression levels of TGFβ did not change regardless of the administration of α-GalCer/DC.However, the phosphorylation of the proteins Smad2 and Smad3 on the downstream side thereof was significantly inhibited by the administration of α-GalCer/DC.

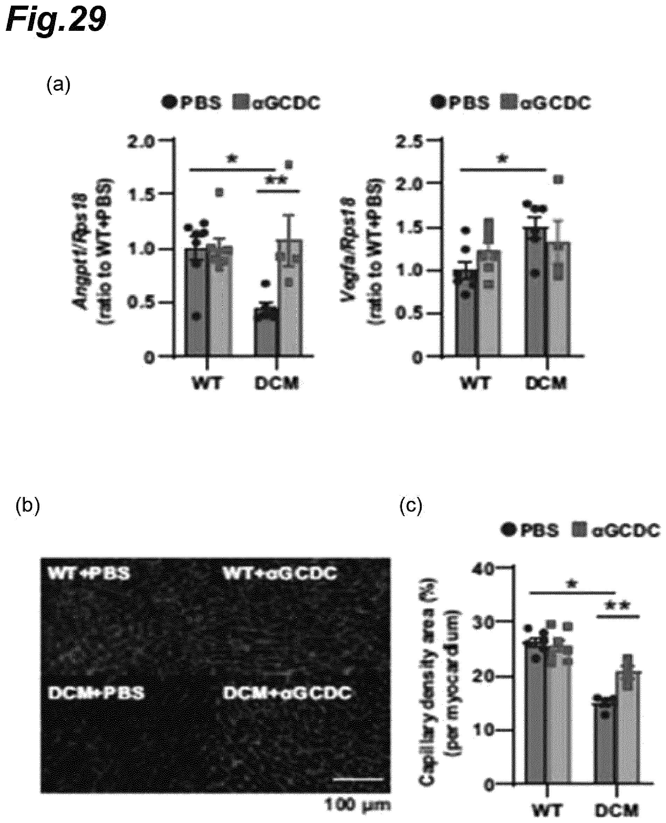

FIG. 29 (a) is a graph showing the expression levels of proteins Angpt1 (angiopoietin-1) and vegfa (vascular endothelial growth factor) in the myocardium of wild mouse (WT) or DCM mouse (DCM) (n = 4 to 6). Angpt1 is a protein involved in angiogenesis. The expression levels of Angpt1 in the myocardium of the DCM mice were inhibited more than the expression levels in the myocardium of the wild mice, but the expression levels of Angpt1 were improved to the same levels as the normal levels (wild mice, α-GalCer non-administration) by administering α-GalCer/DC.Rps18 was used as an intrinsic control. FIG. 29 (b) is a photograph (left) showing the density of capillaries obtained by immunostaining endothelial cells and a graph (right) quantifying the density. As the result of evaluation of the density of capillaries (n = 4 to 7), improvement of the vascular bed was observed due to the administration of α-GalCer/DC.In the graph, ^{∗} represents P < 0.05, and ^{∗∗} represents P < 0.01.

### 9. Evaluation of cell number dependence of α-GalCer/DC administered

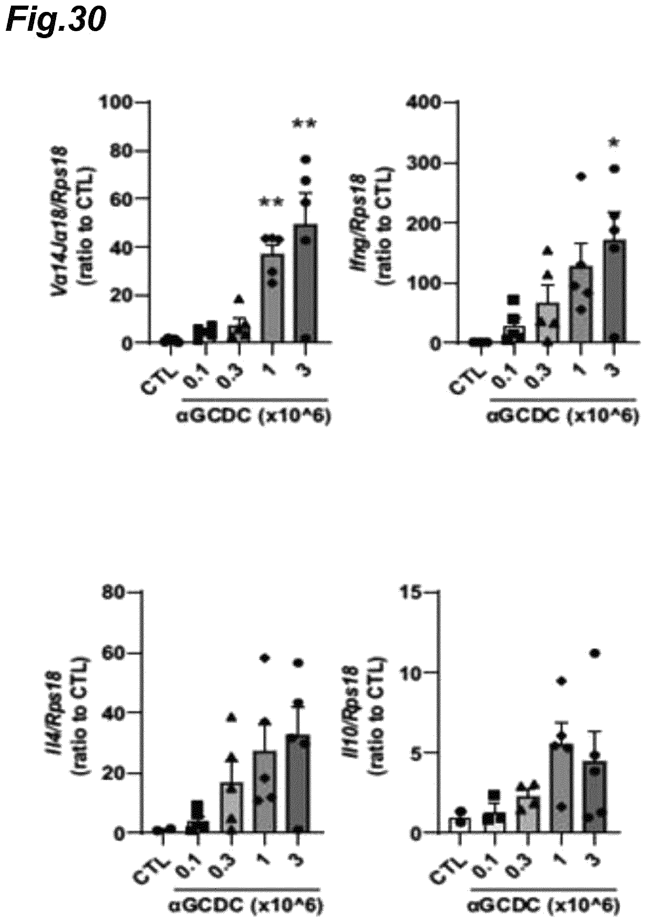

The number of cells of α-GalCer/DC administered was changed, and the influence of the change in number on the expression levels of genes in the myocardium was evaluated. FIG. 30 is a graph showing the expression level of each gene 4 days after administration of α-GalCer/DC to Balb/c mice (each n = 5). Rps18 was used as an intrinsic control. When α-GalCer/DC was administered, the expression levels of cytokine genes (Vα14Jα18 (invariant TCR), Ifng, IL4, IL10) in the myocardium were increased depending on the number of administered cells.

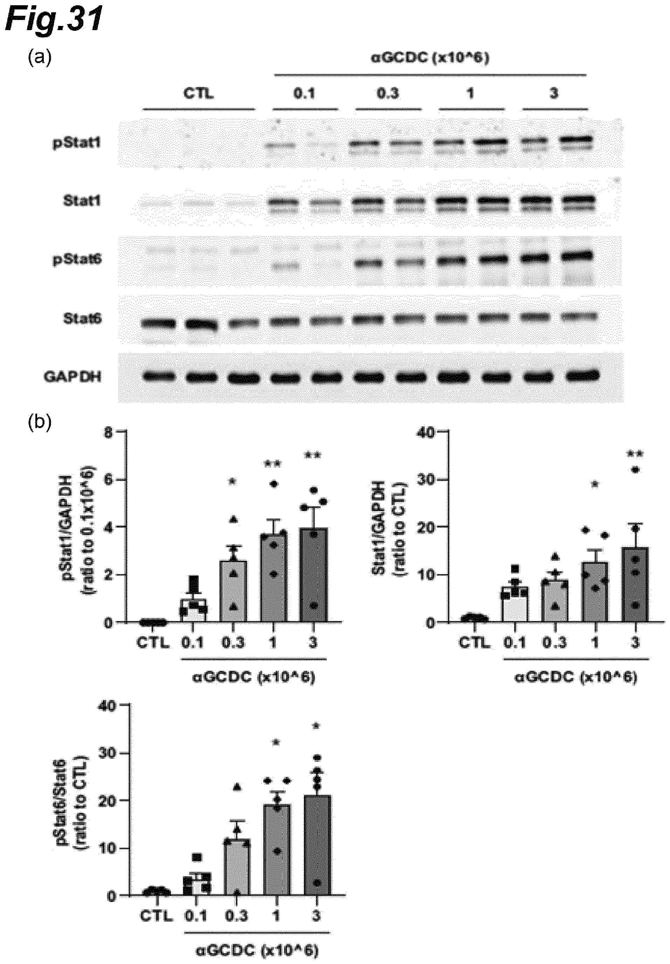

FIG. 31 (a) is a western blot showing the expression levels of proteins (pStat1, Stat1, pStat6, and Stat6) in the myocardium (n = 3 to 8). pStat1 and pStat6 each indicates phosphorylated Stat1 and phosphorylated Stat6. GAPDH was used as an intrinsic control. FIG. 31 (b) is a graph quantifying the results of FIG. 31 (a). As the number of cells of α-GalCer/DC administered was increased, the expression level of Stat1 and the phosphorylation of Stat1 were increased, and the phosphorylation of Stat6 was also promoted. In the graph, ^{∗} represents P < 0.05, and ^{∗∗} represents P < 0.01.

### 10. Difference between α-GalCer administration and α-GalCer/DC administration

The differences in expression levels of proteins Stat1 and Stat6 and degrees of phosphorylation in a case where only α-GalCer was administered and in a case where DC was administered as a carrier (that is, α-GalCer/DC was administered, the number of administered cells: 3 × 10⁶) were evaluated by western blotting.

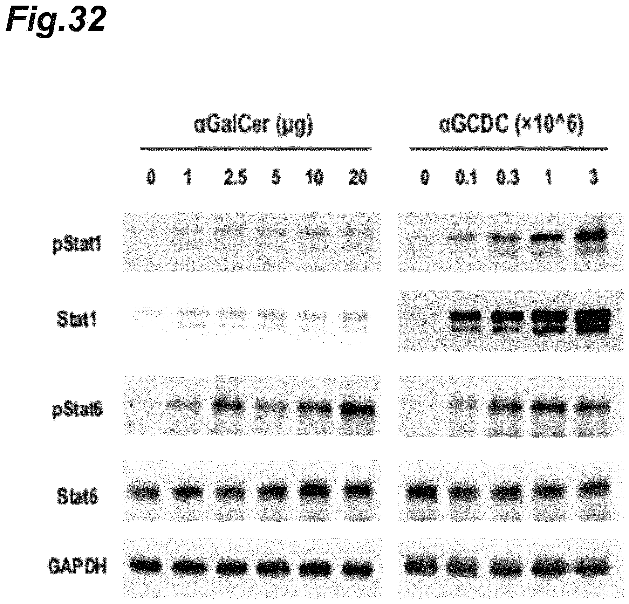

In the test, α-GalCer alone or α-GalCer/DC was administered to the Balb/c mice, and the expression level of each protein in the myocardium 4 days after the administration was evaluated by western blotting. The results are shown in FIG. 32. In a case where only α-GalCer was administered, the activation reaction of Stat1 was extremely weak and dose dependence was not observed.

### 11. Changes over time after administration of α-GalCer/DC

The changes of Stat phosphorylation in the myocardium over time after administration of α-GalCer/DC (number of administered cells: 3 × 10⁶) were evaluated. In the test, α-GalCer/DC was administered to the Balb/c mice once, and the expression level of each protein (pStat1, Stat1, pStat6, Stat6, pStat3, and Stat3) was measured on the day of administration (Day 0) and 1, 4, 7, 14, and 28 days after the day of administration (Days 1, 4, 7, 14, and 28 in order). pStat3 indicates phosphorylated Stat3. GAPDH was used as an intrinsic control.

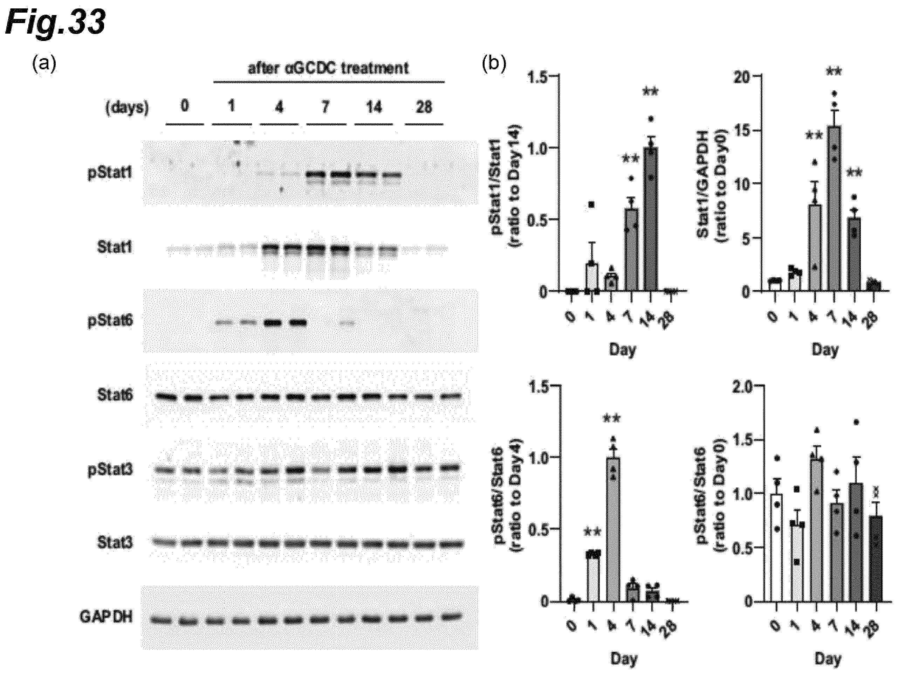

The results are shown in FIG. 33. FIG. 33 (a) is a western blot showing changes in the expression level of each protein over time (each n = 4). FIG. 33 (b) is a graph quantifying the results of FIG. 33 (a). The horizontal shaft represents the number of days after administration of α-GalCer/DC. The expression level of Stat1 began to increase from Day 4, and the expression of Stat1 and the phosphorylation of Stat1 peaked on Days 7 to 14. Meanwhile, the phosphorylation of Stat6 peaked on Day4. Thereafter, the expression levels of Stat1, pStat1, and pStat6 were significantly decreased. In the graph, ^{∗} represents P < 0.05 and ^{∗∗} represents P < 0.01, which are at significant levels with respect to the expression level on Day 0.

### 12. Changes in expression levels of cytokine genes due to α-GalCer/DC administration

The expression levels of cytokine genes (IFNγ, IL4, and IL10) in the myocardium 4 days after administration of PBS or α-GalCer/DC (number of administered cells: 3 × 10⁶) to wild mice (WT) or DCM mice (DCM) were evaluated.

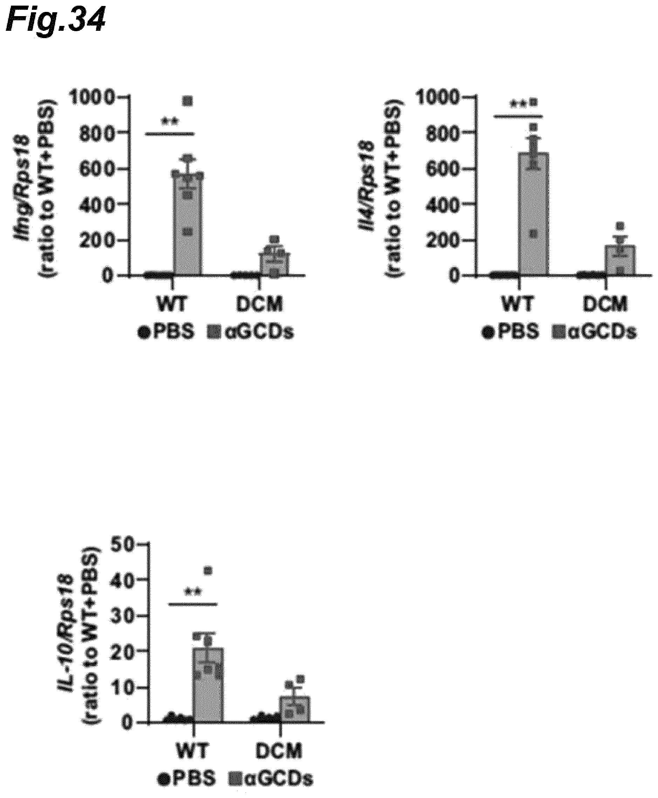

FIG. 34 is a graph showing the expression levels of cytokine genes (Ifng, IL4, and IL10) in the myocardium. Rps18 was used as an intrinsic control. In a case where α-GalCer/DC was administered to wild mice, the expression levels of all cytokine genes were significantly increased. Further, even the expression of cytokine genes in the DCM mice was significantly increased even though the expression was weaker than that in wild mice.

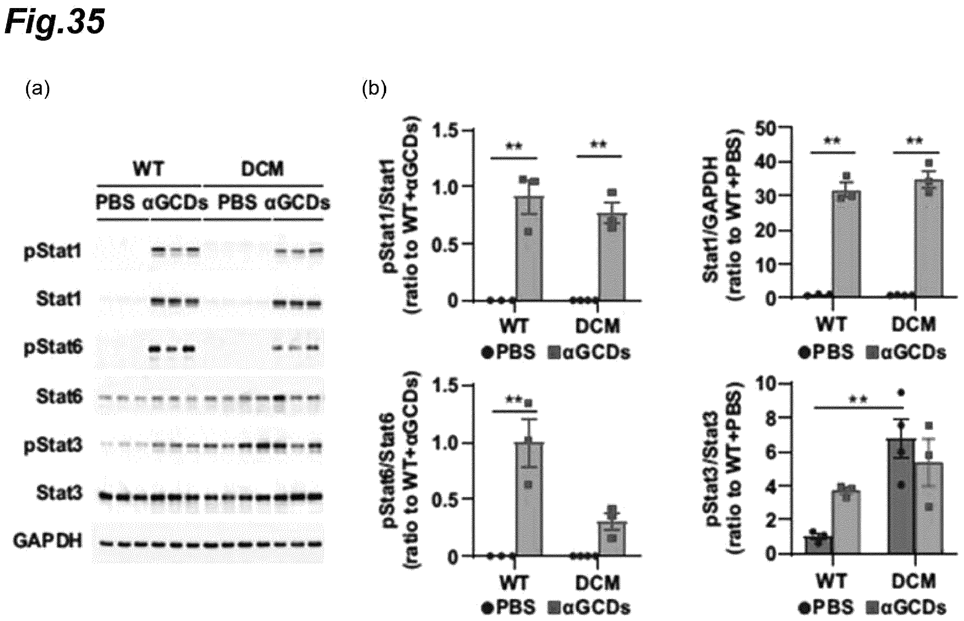

FIG. 35 (a) is a western blot showing the expression level of each protein (pStat1, Stat1, pStat6, Stat6, pStat3, and Stat3) (each n = 3). FIG. 35 (b) is a graph quantifying the results of FIG. 35 (a). GAPDH was used as an intrinsic control. The expression of Stat1 and the phosphorylation of Stat1 and Stat6 were significantly increased due to the administration of α-GalCer/DC to both wild and DCM mice. In the graphs, ^{∗∗} represents P < 0.01. In regard to the phosphorylation of Stat3, the proportion of pStat3 in wild mice was increased and the proportion of pStat3 in DCM mice was decreased due to the administration of α-GarCer/DC.

### 13. Changes in expression levels of cytokine genes due to α-GalCer/DC administration

The expression levels of cytokine genes (Vα14Jα18, Ifng, IL4, and IL10) in the myocardium were evaluated 28 days after the administration of α-GalCer/DC (number of administered cells: 3 × 10⁶) to wild mice (WT) or DCM mice (DCM).

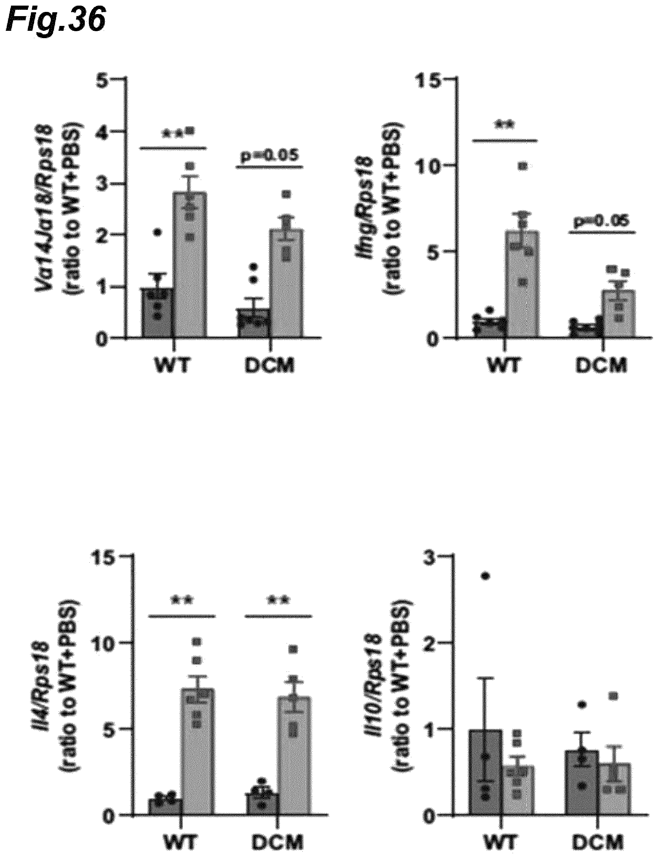

FIG. 36 is a graph showing the expression levels of cytokine genes (Vα14Jα18, Ifng, IL4, and IL10) in the myocardium. In the graphs, ^{∗∗} represents P < 0.01. The expression of genes of Vα14Jα18, Ifng, and IL4 was significantly increased even 28 days after the administration of α-GalCer/DC, but the expression of IL10 was not changed as compared with that in the wild mice. Based on comparison with the results of Day 4 shown in FIG. 34, the increase of the cytokine genes on Day 28 was small.

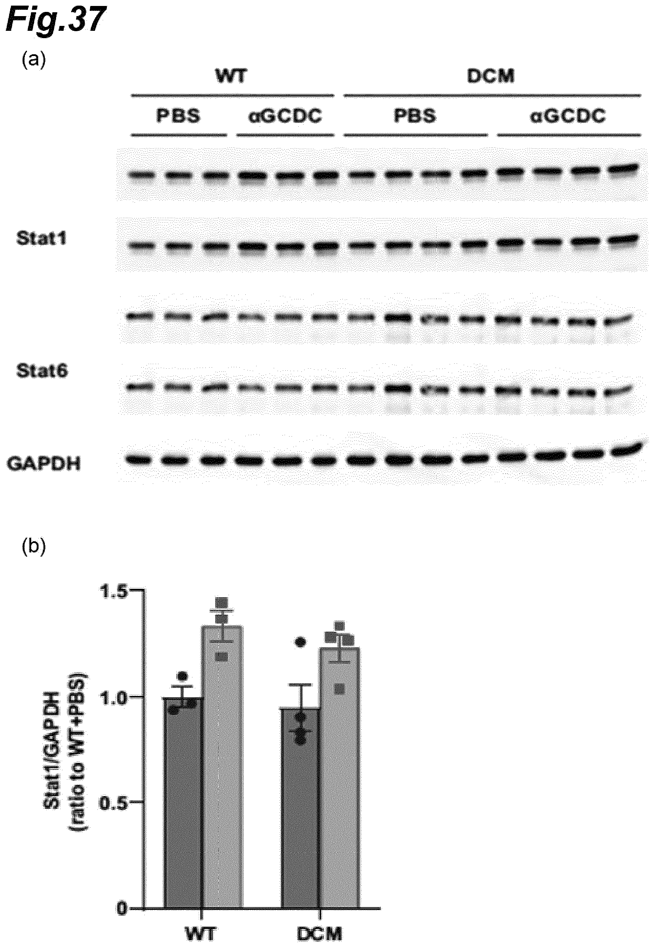

FIG. 37(a) is a western blot showing the expression levels of proteins (Stat1 and Stat6) 28 days after the administration of PBS or α-GalCer/DC (Day 28). GAPDH was used as an intrinsic control. On Day 28, no significant difference was observed in the expression levels of Stat1 between wild mice and DCM mice due to the administration of α-GalCer/DC.

### 14. A-GalCer treatment of myocardial fibroblasts

The changes in expression levels of genes (Ctgf, Col1a, and Col3a) involved in fibrosis after IFNγ, IL4, or IL10 was administered to primary cultured myocardial fibroblasts were evaluated in order to investigate the mechanism by which the TGFβ-Smad signaling system was inhibited due to the administration of α-GalCer/DC (number of administered cells: 3 × 10⁶).

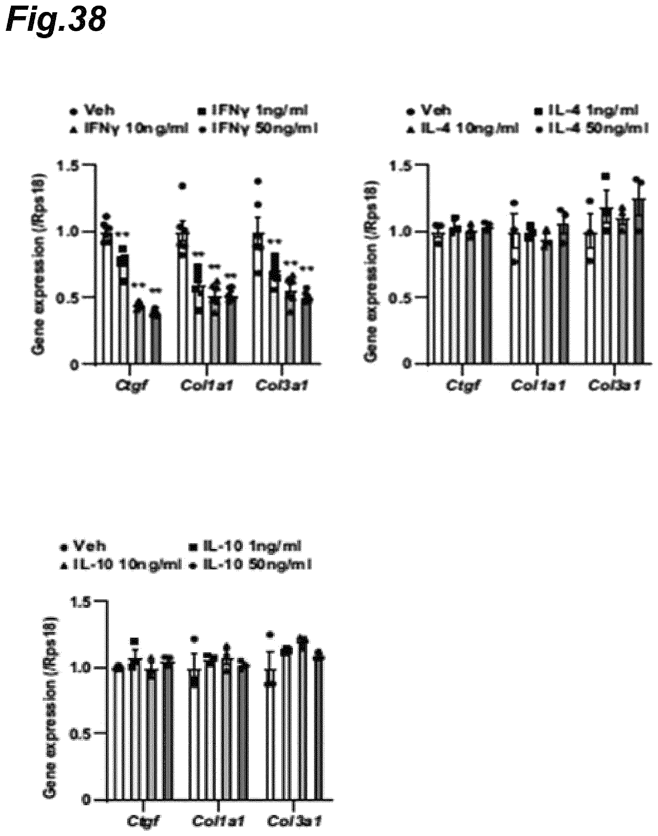

FIG. 38 is a graph showing changes in the expression levels of genes (Ctgf, Col1a, and Col3a) after the administration of IFNγ, IL4, or IL10 to myocardial fibroblasts. IFNγ, IL4, and IL10 were respectively administered at concentrations of 1, 10, and 50 ng/mL. Rps18 was used as an intrinsic control. "Veh" in the graphs represents a non-administration group (Vehicle). The IL4 and IL10 administration groups showed the expression levels of genes which were the same as those in the non-administration group at all concentrations. Meanwhile, the expression levels of genes in the IFNγ administration group were significantly decreased in a dose-dependent manner. The results suggest that IFNγ is a factor that inhibits the TGFβ-Smad signaling system.

### 15. Effect of pretreatment with IFNγ

The changes in expression levels of proteins when TGFβ was administered to myocardial fibroblasts pretreated with IFNγ were evaluated.

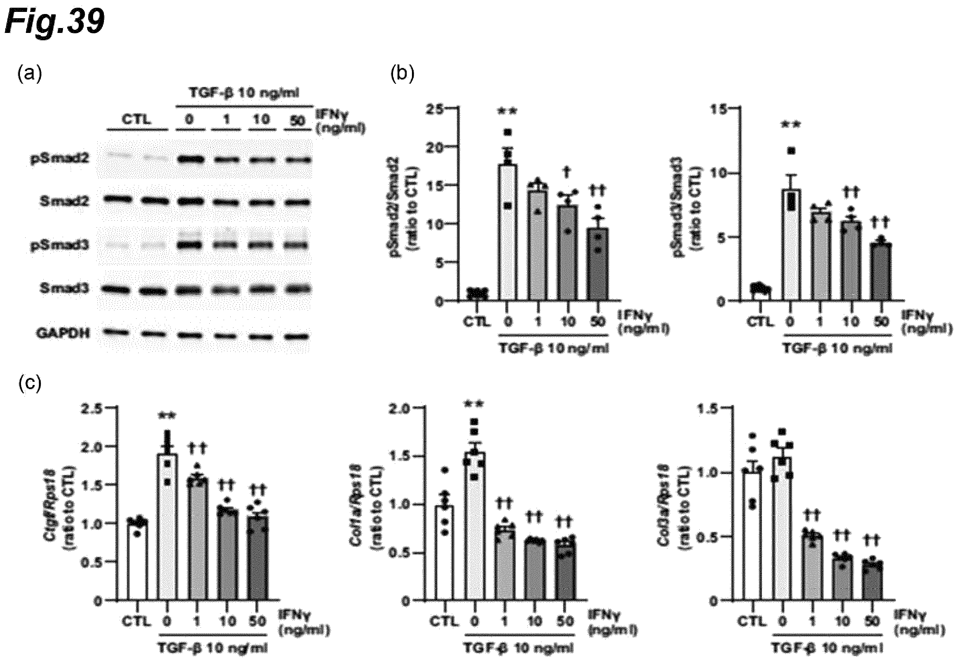

FIG. 39 (a) is a western blot showing the expression levels of proteins (pSmad2, Smad2, pSmad3, and Smad3) when TGFβ was administered to myocardial fibroblasts pretreated with IFNγ (n = 4). FIG. 39 (b) is a graph quantifying the results of FIG. 39 (a). The dose of IFNγ used for the pretreatment is 0, 1, 10, or 50 ng/mL, and the dose of TGFβ is 10 ng/mL. In the control group (CTL), neither IFNγ nor TGFβ was administered. Each graph is shown as a relative value in which the phosphorylation ratio (for example, pSmad2/Smad2) in the CTL group is set to 1.0. When TGFβ was administered, Smad2 and Smad3 were phosphorylated, and the expression levels of pSmad2 and pSmad3 were increased. When the pretreatment was carried out with IFNγ, the phosphorylation ratio of Smad2 and Smad3 was decreased in a dose-dependent manner.

FIG. 39 (c) is a graph showing the expression levels of proteins (Ctgf, Col1a, and Col3a) when TGFβ was administered to myocardial fibroblasts pretreated with IFNγ (n = 3 to 6). The dose of IFNγ used for the pretreatment is 0, 1, 10, or 50 ng/mL, and the dose of TGFβ is 10 ng/mL. Each graph is shown as a relative value in which the phosphorylation ratio (for example, pSmad2/Smad2) in the CTL group is set to 1.0. In the graph, ^{∗∗} represents P < 0.01, which is at a significant level with respect to the expression level of the CTL group. In the graph, † represents P < 0.05 and †† represents P < 0.01, which are at significant levels with respect to the expression levels of the TGFβ administration group that was not pretreated with IFNγ. When TGFβ was administered, the expression levels of the above-described proteins were increased. In addition, when the pretreatment was carried out with IFNγ, the increase in the expression levels due to the administration of TGFβ was inhibited in a dose-dependent manner of IFNγ.

### 16. Treatment with IFNγ, IL4, or IL10

The primary cultured myocardial fibroblasts were treated with IFNγ, IL4, or IL10, and each of the expression levels of proteins (Stat1, Stat6, and Stat3) and the phosphorylation thereof were evaluated.

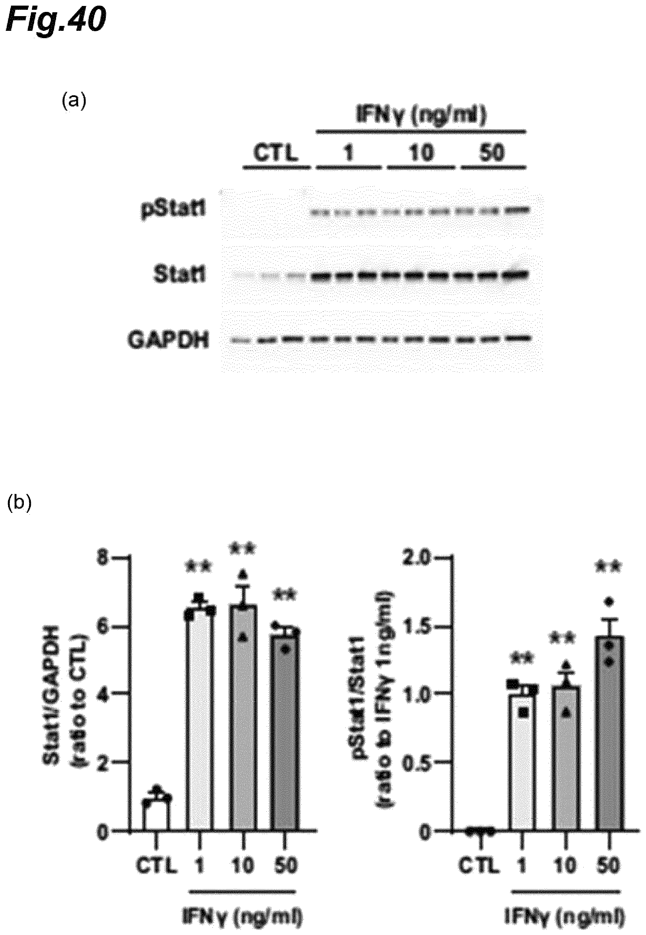

FIG. 40 (a) is a western blot showing the expression levels of Stat1 and the phosphorylation ratio when myocardial fibroblasts were treated with IFNγ. FIG. 40 (b) is a graph quantifying the results of FIG. 40 (a), the graph on the left shows the expression level of Stat1, and the graph on the right shows the phosphorylation state of Stat1. GAPDH was used as an intrinsic control. In the graph, ^{∗∗} represents P < 0.01, which is at a significant level with respect to the expression level of the CTL group. When IFNγ was administered to myocardial fibroblasts, both the expression levels and phosphorylation of Stat1 were increased.

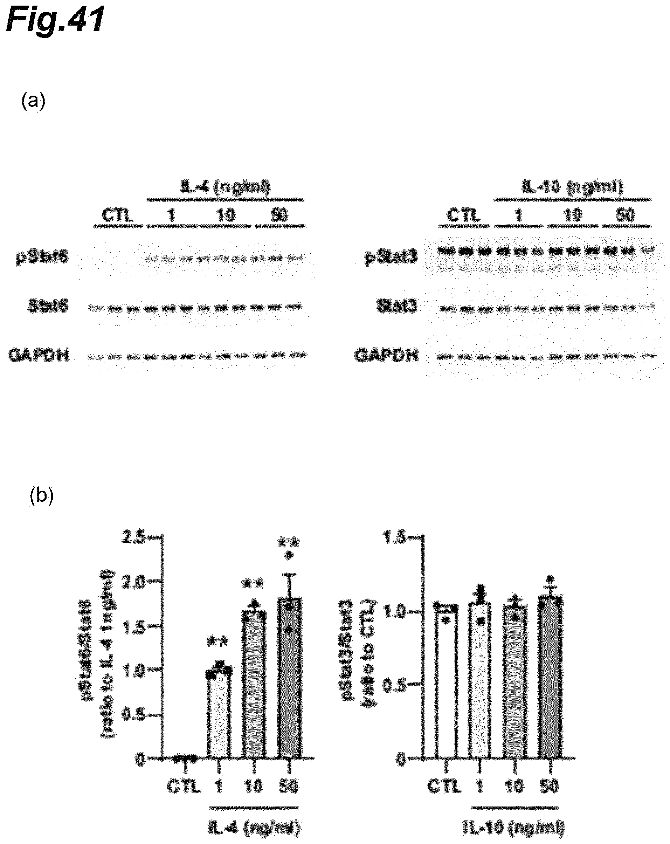

FIG. 41 (a) is a western blot showing the expression levels and phosphorylation ratio of Stat6 or Stat3 when myocardial fibroblasts were treated with IL4 or IL10. FIG. 41 (b) is a graph quantifying the results of FIG. 41 (a) and shows the phosphorylation state of Stat6 or Stat3. In the graph, ^{∗∗} represents P < 0.01, which is at a significant level with respect to the expression level of the CTL group. When IL4 was administered to myocardial fibroblasts, the phosphorylation of Stat6 was increased in a dose-dependent manner. When IL10 was administered to myocardial fibroblasts, a change in the phosphorylation of Stat6 was not found.

### 17. Changes in expression levels of Angpt1 due to cytokine administration

The changes in the expression levels of an angiogenic factor Angptl after administration of IFNγ, IL4, or IL10 to offspring rat myocardial cells were evaluated (each n = 3).

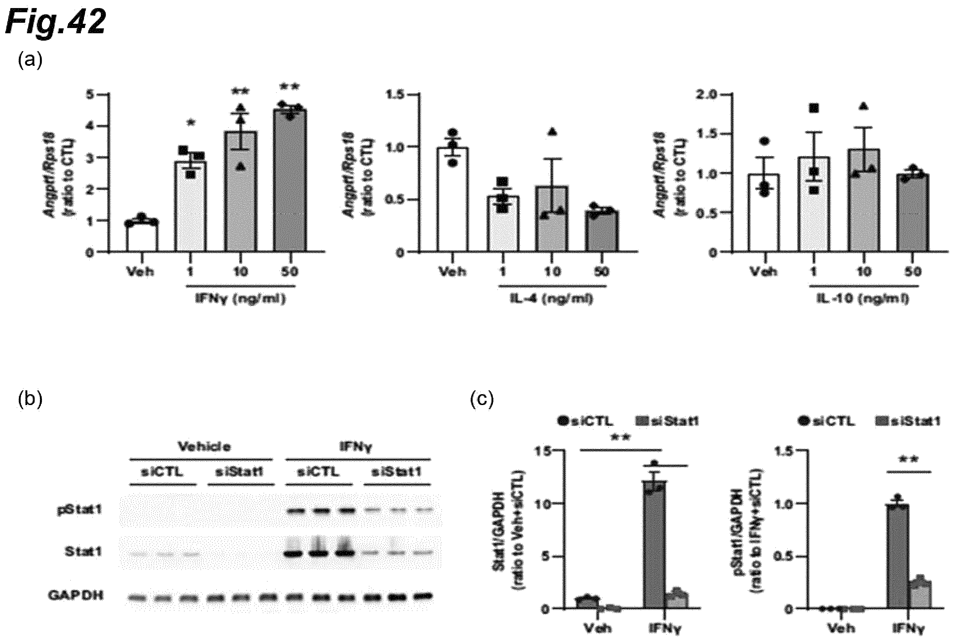

FIG. 42 (a) is a graph showing changes in the expression levels of the angiogenic factor Angptl after administration of IFNγ, IL4, or IL10 to offspring rat myocardial cells. In the graphs, ^{∗} represents P < 0.05 and ^{∗∗} represents P < 0.01, which are at significant levels with respect to the expression levels of the CTL group. When IFNγ was administered, the expression levels of Angpt1 were increased in a dose-dependent manner. On the contrary, the expression levels of Angpt1 were not increased even when IL4 or IL10 was administered.

### 18. Effects of Stat1 targeted siRNA

The primary cultured myocardial cells were transfected with Stat1 targeting siRNA, and the total expression levels of Stat1 and the state of phosphorylation thereof when IFNγ (50 ng/mL) was administered were evaluated. In the control group (siCTL), myocardial cells were transfected with scrambled siRNA derived from Stat1 targeting siRNA.

FIG. 42 (b) is a western blot showing the expression levels of pStat1 and Stat1 (each n = 3). GAPDH was used as an intrinsic control. In the graphs, ^{∗∗} represents P < 0.01, which is at a significant level with respect to the expression level of the siCTL group. FIG. 42 (c) is a graph quantifying the results of FIG. 42 (b). The knockout with Stat1 targeting siRNA significantly inhibited the increase in expression levels of Stat1 and pStatl due to the administration of IFNγ.

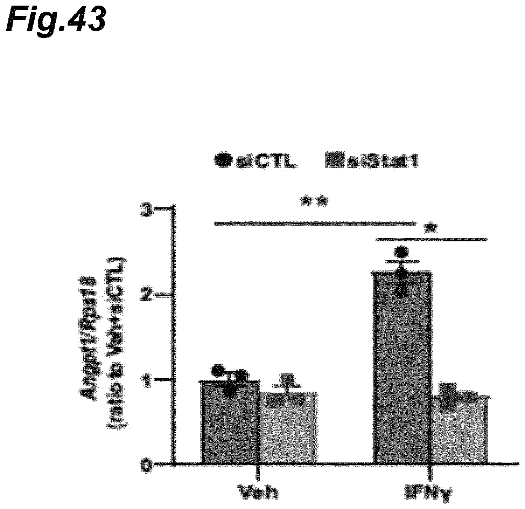

FIG. 43 is a graph showing changes in expression levels of Angptl due to administration of IFNγ. In the graphs, ^{∗} represents P < 0.05 and ^{∗∗} represents P < 0.01, which are at significant levels with respect to the expression levels of the Veh + siCTL group (IFNγ was not administered to the mice of the siCTL group). The knockout with Stat1 targeting siRNA significantly inhibited the increase in expression levels of Angptl due to the administration of IFNγ.

### 19. Administration of IFNγ, IL4, or IL10 to primary cultured offspring rat myocardial cells

IFNγ, IL4 or IL10 was administered to primary cultured offspring rat myocardial cells, and the changes in phosphorylation of proteins (Stat1, Stat6, and Stat3) were evaluated.

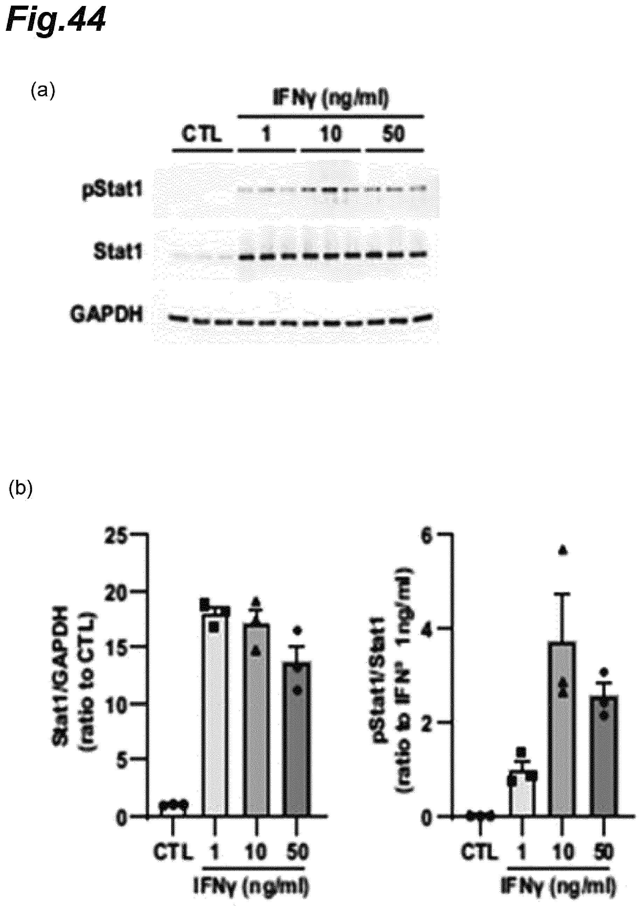

FIG. 44 (a) is a western blot showing phosphorylation of Stat1 after administration of IFNγ to primary cultured myocardial cells. FIG. 44 (b) is a graph quantifying the results of FIG. 44 (a). GAPDH was used as an intrinsic control. In the graph, ^{∗} represents P < 0.05, which is at a significant level with respect to the expression levels of the CTL group.

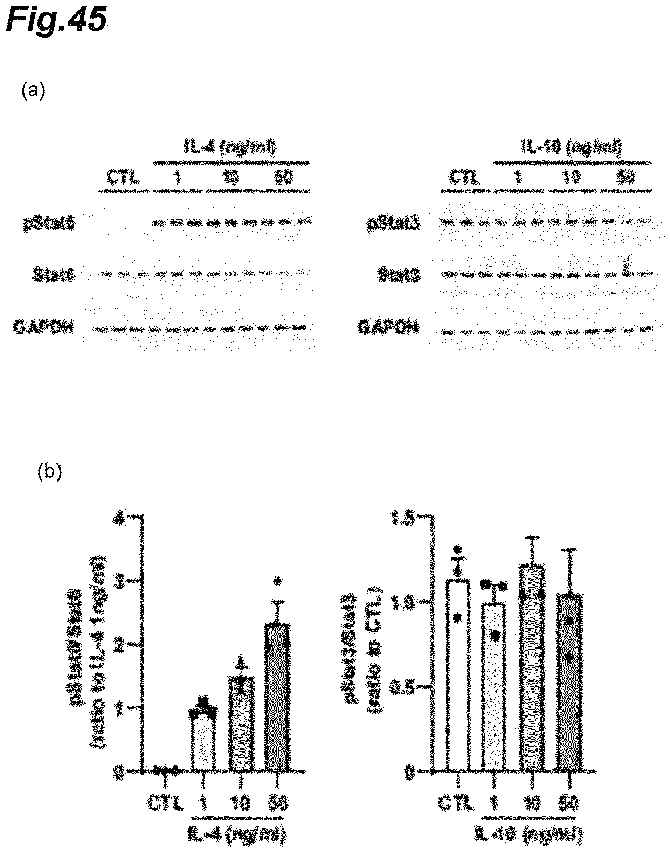

FIG. 45 (a) is a western blot showing phosphorylation of Stat6 or Stat3 after administration of IL4 or IL10 to primary cultured myocardial cells. FIG. 45 (b) is a graph quantifying the results of FIG. 45 (a). In the graph, ^{∗} represents P < 0.05, which is at a significant level with respect to the expression levels of the CTL group. The phosphorylation of Stat1 by αGalCer/DC observed in myocardial tissues was reproduced in cultured myocardial cells due to the administration of IFNγ and IL-4, but was not reproduced when IL-10 was administered. That is, it is considered that the cytokines involved in the phosphorylation of Stat1 due to αGalCer/DC may be IFNγ and IL-4.

### 20. Administration of α-GalCer/DC to patient with chronic heart failure

The patients with cardiomyopathy usually have physical restrictions (shortness of breath, fatigue, and swelling of ankles) and mental restrictions (depression and anxiety) due to impaired cardiac function. Therefore, in general, relaxation of these restrictions leads to improvement of the patient's quality of life (QOL) and can be used as an index for secondary evaluation of the effect of treating cardiomyopathy. Therefore, α-GalCer/DC was administered twice to 5 patients with chronic heart failure whose ejection fractions were decreased. As for the administration method, the number of administered cells per dose was set to 2.5 × 10⁸ cells/m², and the cells were intravenously infused twice at 6-day intervals. The patient's quality of life (QOL) was evaluated four weeks after the administration using the Minnesota Living with Heart Failure Questionnaire (MLHFQ) and the Kansas City Cardiomyopathy Questionnaire (KCCQ).

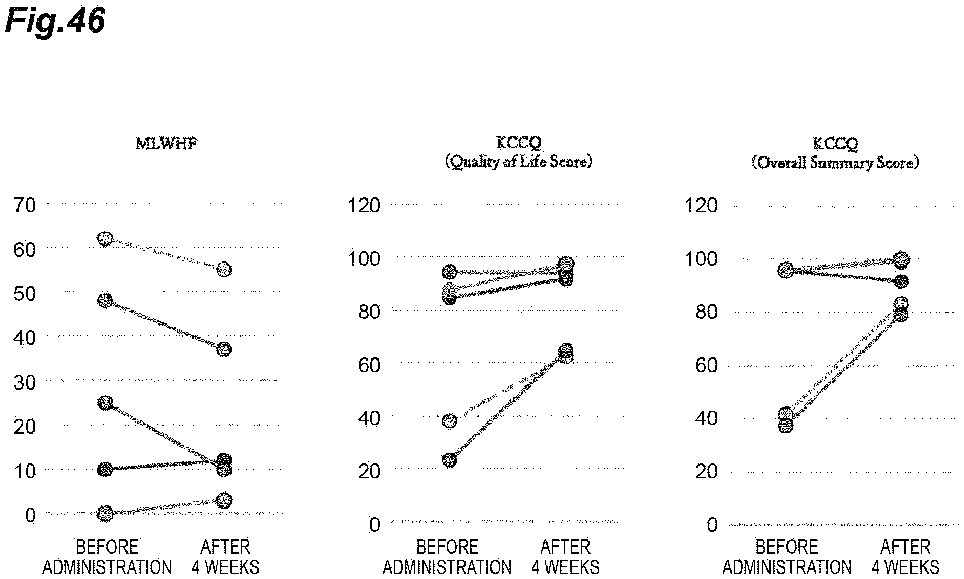

In the evaluation based on MLHFQ (Heart Fail. 1987; 3: 198-209), there are 21 questions, and each question is scored on a 6-point scale from 0 to 5. As the QOL is higher, the total score decreases. The results are shown in FIG. 46. The score before administration was 29.0 ± 25.8, but the score after 4 weeks was decreased to 23.4 ± 21.8, which shows a significant improvement tendency (p = 0.189, paired t-test). In particular, among the 5 subjects, 3 subjects whose QOL was originally low showed a significant improvement in the score.

In the evaluation based on KCCQ (J. Am. Coll. Cardiol. 35: 1245-5 (2000)), the score was calculated according to The Kansas City Cardiomyopathy Questionnaire Scoring Instructions. As the QOL is higher, the total score increases. The results are shown in FIG. 46. The score before administration was 50.0 ± 24.30, but the score after 4 weeks was increased to 66.67 ± 24.30, which shows a significant improvement tendency (p = 0.003, paired t-test). In regard to the overall summary score, the score before administration was 64.79 ± 31.58, but the score after 4 weeks was improved to 82.05 ± 17.02 (p = 0.044, paired t-test).

It was shown that the QOL of patients with heart failure was significantly improved due to the administration of αGalCer/DC, which is considered that the patient's subjective symptoms were improved as a result of the improvement of the heart function, and this is the result supporting the result obtained from the animal experiments carried out so far.

### 21. Inhibition of infiltration of inflammatory cells in myocardium

Administration of IFNγ to inflammatory cells easily caused cell death, but administration of IFNγ to myocardial cells did not cause cell death. It was suggested that infiltration of inflammatory cells was inhibited in DCM myocardium.

## Claims

1. A pharmaceutical for preventing and/or treating non-ischemic cardiomyopathy, comprising:
dendritic cells obtained by a method comprising a step of culturing mononuclear cells in the presence of GM-CSF and IL-2 and a step of pulsing the cultured cells with α-galactosylceramide.

2. The pharmaceutical according to claim 1, wherein the non-ischemic cardiomyopathy is dilated cardiomyopathy or drug-induced cardiomyopathy caused by an anthracycline-based anticancer agent.

3. The pharmaceutical according to claim 1 or 2, further comprising an anthracycline-based anticancer agent.

4. The pharmaceutical according to any one of claims 1 to 3, wherein the pharmaceutical is used for preventing and/or treating cardiomyopathy of a subject to whom an anthracycline-based anticancer agent has been administered.

5. A method of producing a pharmaceutical for preventing and/or treating non-ischemic cardiomyopathy, comprising:
a step of culturing mononuclear cells in the presence of GM-CSF and IL-2; and
a step of pulsing the cultured cells with α-galactosylceramide.

6. The method of producing a pharmaceutical according to claim 5, wherein the non-ischemic cardiomyopathy is dilated cardiomyopathy or drug-induced cardiomyopathy caused by an anthracycline-based anticancer agent.

7. A pharmaceutical for inhibiting a decrease in myocardial contractility, the pharmaceutical comprising:
dendritic cells obtained by a method comprising a step of culturing mononuclear cells in the presence of GM-CSF and IL-2 and a step of pulsing the cultured cells with α-galactosylceramide.

8. A pharmaceutical for inhibiting or preventing myocardial cell death, the pharmaceutical comprising:
dendritic cells obtained by a method comprising a step of culturing mononuclear cells in the presence of GM-CSF and IL-2 and a step of pulsing the cultured cells with α-galactosylceramide.

9. A pharmaceutical for inhibiting or improving myocardial atrophy, the pharmaceutical comprising:
dendritic cells obtained by a method comprising a step of culturing mononuclear cells in the presence of GM-CSF and IL-2 and a step of pulsing the cultured cells with α-galactosylceramide.

10. A pharmaceutical for progress-inhibiting and/or treating myocardial fibrosis, the pharmaceutical comprising:
dendritic cells obtained by a method comprising a step of culturing mononuclear cells in the presence of GM-CSF and IL-2 and a step of pulsing the cultured cells with α-galactosylceramide.
